# EUROPEAN PATENT SPECIFICATION

(11) **EP 3 406 586 B1**
(45) Date of publication and mention of the grant of the patent: **30.12.2020**
(21) Application number: 17741118.8
(22) Date of filing: 22.01.2017
(51) Int. Cl.: C07C 249/16, C07C 45/42, C07C 251/84, C07C 49/713, C07C 49/753, C07C 311/49

(54) **MANUFACTURING METHOD FOR HIGH-PURITY CYCLOHEXENONE LONG-CHAIN ALCOHOL**
HERSTELLUNGSVERFAHREN FÜR HOCHREINEN CYCLOHEXENON-LANGKETTEN-ALKOHOL
PROCÉDÉ DE FABRICATION D'UN ALCOOL À CHAÎNE LONGUE CYCLOHEXÉNONE DE GRANDE PURETÉ

(30) Priority: 22.01.2016 CN 201610044990
(43) Date of publication of application: 28.11.2018
(73) Proprietor: Taiho Pharmaceutical Co., Ltd., Chiyoda-ku, Tokyo 101-8444 (JP)
(72) Inventor: ZHANG, Jian, Shanghai 201209 (CN); JIANG, Dehui, Shanghai 201209 (CN); SHEN, Xiaojun, Shanghai 201209 (CN)
(74) Representative: Hoffmann Eitle
(86) International application number: PCT/CN2017/072078
(87) International publication number: WO 2017/125087

(56) References cited:
- WO-A1-2004/087630
- FARHAD SHIRINI ET AL: "Efficient oxidative cleavage of C=N using chromium trioxide supported on NaHSO 4 . H 2 O", ARKIVOC, 1 December 2007 (2007-12-01), XP055532363,
- HUBER, TATJANA et al.: "Experimental Studies on the Selective beta -C-H Halogenation of Enones", The Journal of Organic Chemistry, vol. 80, no. 4, 26 January 2015 (2015-01-26), pages 2281-2294, XP055400738, ISSN: 0022-3263
- SHIRINI, FARHAD et al.: "Efficient Oxidative Cleavage of C=N Using Chromium Trioxide Supported on NaHSO4.H2O", Arkivoc, 31 December 2007 (2007-12-31), pages 34-39, XP055532363, ISSN: 1424-6376
- LEVINE, SAMANTHA R. et al.: "Catalytic Enantioselective Approach to the Eudesmane Sesquiterpenoids: Total Synthesis of (+)-Carissone", Organic Letters, vol. 11, no. 2, 15 January 2009 (2009-01-15), pages 289-292, XP055532366, ISSN: 1523-7060
- LUMB, ANSHIKA et al.: "A Simple, Mild and Environmentally Benign Procedure for the Cleavage of Carbon-Nitrogen Double Bonds Using NaBr03 in the Presence of [Bmim] HS04", Royal Society of Chemistry Advances, vol. 4, 17 September 2014 (2014-09-17), pages 47677-47689, XP055400751, ISSN: 2046-2069
- KIM, SANG-WON et al.: "Vinylogous Intramolecular Nucleophilic Acyl Substitution Reactions Mediated by Samarium(II) Iodide", Bulletin of the Korean Chemical Society, vol. 21, no. 12, 25 August 2000 (2000-08-25), pages 1175-1176, XP055532368, ISSN: 0253-2964

## Description

### Technical Field

The present invention pertains to the fields of pharmacochemistry and synthetic chemistry, and specifically relates to a method for producing a high-purity cyclohexenone long-chain alcohol.

### Background Art

Nerves growth factor (NGF) is present primarily in the hippocampus and the area of the cerebral cortex, playing a role in the regulation of survival, growth and development, differentiation, regeneration, and functional maintenance of neurons. NGF acts not only on catecholaminergic neurons in the peripheral nervous system, but also on cholinergic neurons in the brain. Alzheimer's disease is believed to be associated with degeneration and loss of cholinergic neurons. Researchers once attempted to treat Alzheimer's disease by administering NGFs into the brain, but this type of therapeutic approach was unfit for humans because NGF is a macromolecular protein with a molecular weight of as high as 12000, which is unable to permeate the blood-brain barrier. Thus, researchers have devoted continuous effort to search for an NGF-like substance that can penetrate through the blood-brain barrier or a small-molecule compound that can stimulate NGF synthesis in the brain, and the use of such substances in the treatment of Alzheimer's disease. Long-chain aliphatic alcohols, such as cyclohexenone long-chain alcohols, are classified as small molecules that have a similar nature to that of NGF, and can stimulate the growth of neurons in the brain, showing promise in clinical application.

Literature, Molecules (2000, 5, 1439 to 1460), reports a method for producing a cyclohexenone long-chain alcohol as shown in scheme 1.

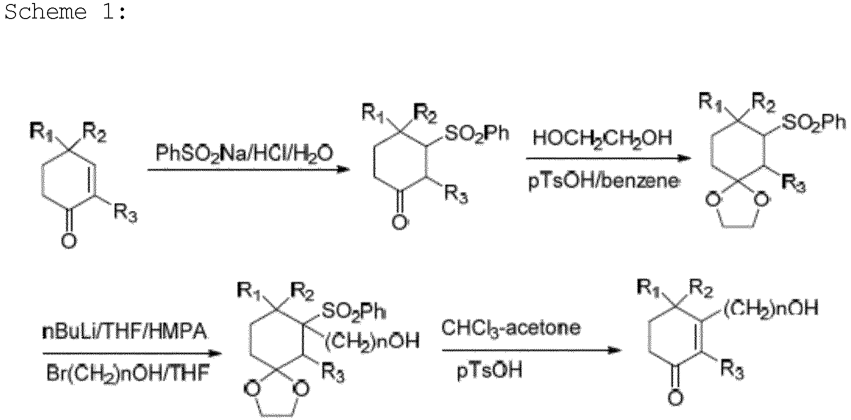

This method is disadvantageous in industrial production because unsaturated cyclohexanone, which is a starting material in scheme 1, is difficult to prepare; the total yield percentage of the method is low; the method uses butyllithium as a transmetalation reagent; and multiple type I solvents (which refer to human carcinogens and organic solvents suspected as human carcinogens or environmentally damaging substances) are involved in the scheme.

Literature, Bioorganic & Medicinal Chemistry Letters (2000, 10, 2537 to 2539), reports a production method shown in scheme 2.

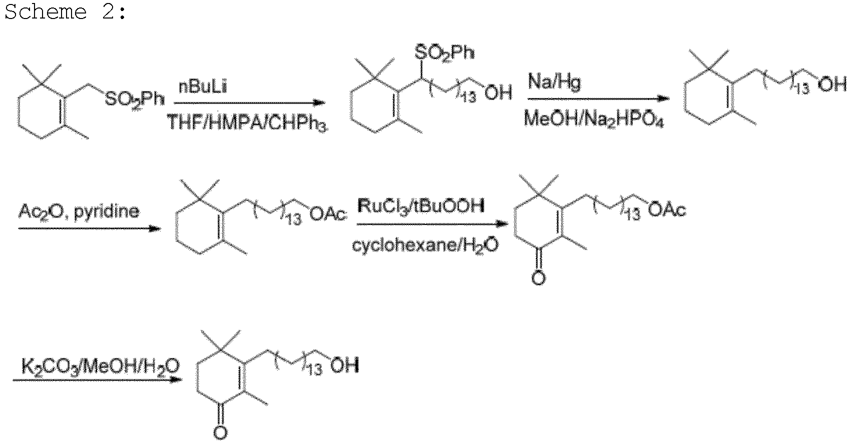

This method is disadvantageous in industrial production because sulfone, which is a starting material in scheme 2, is difficult to prepare; when the sulfone group is removed, highly toxic Na(Hg) needs to be used; and when the carbonyl group is introduced, ruthenium, a very expensive metal, and tert-butyl hydroperoxide, a highly hazardous substance, are used.

WO2004/087630 reports a production method shown in scheme 3.

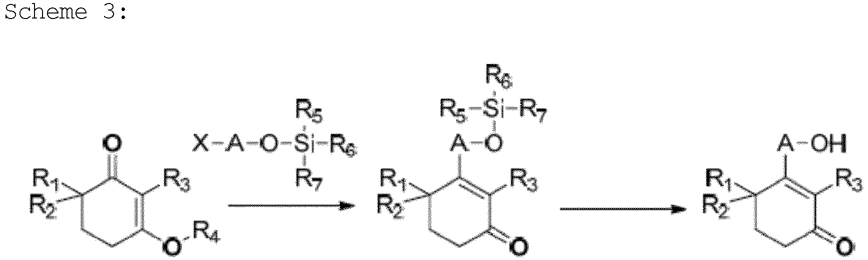

Scheme 3 uses a Grignard reagent to perform a 1,2-addition reaction with an unsaturated ketone. However, the productivity in the addition reaction is merely about 30%, and a large amount of halogenated hydrocarbon protected by silyl ether decomposes during the preparation of the Grignard reagent, considerably increasing the production cost. Additionally, the low productivity and the large amount of by-products generated in the preparation of the Grignard reagent make it extremely difficult to purify the product. Thus, this scheme is also not suitable for industrial production.

Moreover, preparation of a high-purity starting material drug is a major requirement in the development of a cyclohexenone long-chain alcohol as a medicinal substance and its clinical application. Cyclohexenone long-chain alcohols have a low melting point, and transform into an oil as the room temperature increases, which makes it difficult to purify them. Cyclohexenone long-chain alcohols reported in the literature above are all prepared into high-purity products by treatment with column chromatography. Since column chromatography is not suitable for industrial production because of the high cost and great loss, there has been a desperate need for a production method for a high-purity cyclohexenone long-chain alcohol that is produced in a short scheme at a high yield, easy to handle, and suitable for industrial production.

ARKIVOC, 31 December 2007, pages 34-39 describes the use of NaHSO_{4·}H₂O - supported CrO₃ for the oxidative cleavage of oximes, hydrazones, semicarbazones and azines to their corresponding carbonyl compounds under mild and completely heterogeneous reaction conditions.

### Summary of Invention

An object of the present invention is to provide a method for producing a high-purity cyclohexenone long-chain alcohol represented by formula I, and the method is achieved by the following reaction scheme: 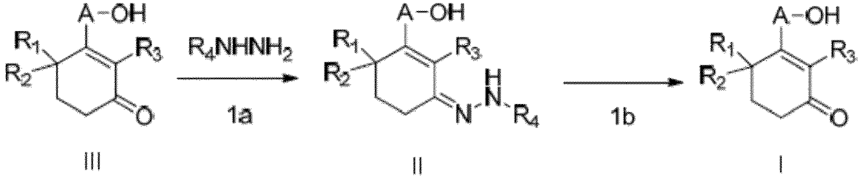 wherein
R₄ represents 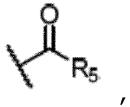
wherein R₅ represents amino,
the method comprising the steps of
   (1a) subjecting a cyclohexenone long-chain alcohol crude product III and hydrazine derivative R₄NHNH₂ to a condensation reaction under suitable conditions to obtain a compound II, and
   (1b) hydrolyzing the compound II in the presence of an acidic substance to obtain a high-purity cyclohexenone long-chain alcohol (compound I).

In this method, the high-purity cyclohexenone long-chain alcohol (compound I) has a purity by HPLC of more than 95%; preferably, the high-purity cyclohexenone long-chain alcohol has a purity by HPLC of more than 99%; and more preferably, the high-purity cyclohexenone long-chain alcohol has a purity by HPLC of more than 99.9%.

In this method, the suitable conditions stated in step (1a) means conditions under which an acid, an alkali, or a desiccant is present. The alkali is one member or two or more members selected from sodium alkoxide, potassium alkoxide, magnesium oxide, calcium oxide, sodium carbonate, potassium carbonate, lithium carbonate, cesium carbonate, calcium carbonate, sodium acetate, potassium acetate, lithium acetate, sodium benzoate, potassium benzoate, lithium benzoate, triethylamine, trimethylamine, diisopropylethylamine, 1,8-diazabicyclo[5.4.0]undec-7-ene, 1,5-diazabicyclo[4.3.0]non-5-ene, and triethylenediamine, and preferably one member or two or more members selected from sodium tert-butoxide, potassium tert-butoxide, sodium carbonate, potassium carbonate, sodium acetate, potassium acetate, triethylamine, and diisopropylethylamine. The acid is one member or two or more members selected from acetic acid, benzoic acid, hydrochloric acid, sulfuric acid, phosphoric acid, benzenesulfonic acid, p-toluenesulfonic acid, camphorsulfonic acid, boron trifluoride ethyl ether, indium trifluoromethanesulfonate, indium trifluoromethanesulfonate, and bismuth trifluoromethanesulfonate, and preferably one member or two or more members selected from acetic acid, p-toluenesulfonic acid, boron trifluoride ethyl ether, and bismuth trifluoromethanesulfonate. The desiccant is one member or two or more members selected from desiccants, such as a molecular sieve, magnesium sulfate, sodium sulfate, and calcium hydride, and preferably one member or two or more members selected from a molecular sieve and magnesium sulfate.

The molar ratio of the hydrazine or its derivative R₄NHNH₂ to the cyclohexenone long-chain alcohol crude product III is 0.8:1 to 3:1, and preferably 0.9:1 to 2:1. The condensation reaction is performed in a solvent, and the solvent is one member or two or more members selected from methanol, ethanol, isopropanol, n-butanol, tert-butanol, tert-pentanol, acetonitrile, tetrahydrofuran, methyl tert-butyl ether, isopropyl ether, dioxane, acetone, 2-butanone, ethyl acetate, isobutyl acetate, toluene, xylene, chlorobenzene, benzene, N,N-dimethylacetamide, N,N-dimethylformamide, N,N-diethylformamide, N-methylpyrrolidone, dichloromethane, 1,2-dichloroethane, chloroform, n-hexane, n-heptane, cyclohexane, and water, and preferably one member or two or more members selected from methanol, ethanol, tetrahydrofuran, acetonitrile, and n-heptane.

The temperature of the condensation reaction is 0 to 149°C, and preferably 20 to 129°C, and the reaction time is 0.5 to 24 hours, and preferably 1 to 10 hours.

In this method, the acidic substance stated in step (1b) is one member or two or more members of an organic acid, an inorganic acid, a Lewis acid, an acid salt, and other acidic substance. The inorganic acid is sulfuric acid, hydrochloric acid, phosphoric acid, polyphosphoric acid, or phosphotungstic acid. The organic acid is formic acid, acetic acid, propionic acid, oxalic acid, fumaric acid, maleic acid, trifluoroacetic acid, methanesulfonic acid, benzenesulfonic acid, p-toluenesulfonic acid, camphorsulfonic acid, or trifluoromethanesulfonic acid. The Lewis acid is boron trifluoride ethyl ether, aluminum trichloride, iron trichloride, bismuth trifluoromethanesulfonate, or indium trifluoromethanesulfonate. The acid salt is an acid salt, such as sodium hydrogensulfate, ammonium hydrogensulfate, magnesium hydrogensulfate, pyridinium p-toluenesulfonate, triethylamine hydrochloride, and pyridine hydrochloride. The other acidic substance is silica gel, acidic resin, or acidic resin. Preferably, the acidic substance is p-toluenesulfonic acid, hydrochloric acid, sulfuric acid, sodium hydrogensulfate, or magnesium hydrogensulfate. The molar ratio of the added amount of the compound II to the added amount of the acidic substance is 1:0.2 to 1:10, and preferably 1:0.2 to 1:2.

The hydrolysis reaction is performed in a solvent, and the solvent is one member or two or more members selected from benzene, toluene, chlorobenzene, xylene, acetonitrile, 2-butanone, acetone, 1,2-dimethyl-2-imidazolone, dimethyl sulfoxide, dimethyl sulfone, sulfolane, hexamethylphosphoric triamide, N,N-dimethylformamide, N,N-dimethylacetamide, N,N-diethylformamide, N-methylpyrrolidone, methanol, ethanol, isopropanol, n-butanol, ethylene glycol, polyethylene glycol, dioxane, methyl tert-butyl ether, isopropyl ether, tetrahydrofuran, n-hexane, cyclohexane, dichloromethane, 1,2-dichloroethane, chloroform, and water; and preferably, the solvent is one member or two or more members selected from toluene, acetonitrile, methanol, ethanol, water, tetrahydrofuran, methyl tert-butyl ether, and dichloromethane.

The temperature of the hydrolysis reaction is selected from 20 to 139°C, and the reaction time is 0.5 to 24 hours. Preferably, the reaction temperature is 20 to 100°C, and the reaction time is 0.5 to 10 hours.

The cyclohexenone long-chain alcohol crude product (compound III) refers to such a product on which a purification step has not been performed. When the content of the cyclohexenone long-chain alcohol is 95% or less, the alcohol product is considered to be a crude product. Typically, the content of the cyclohexenone long-chain alcohol crude product (compound III) is 45 to 80% when the method of the present invention is used (HPLC external standard method).

The present invention further provides a method for producing a high-purity cyclohexenone long-chain alcohol represented by formula I, achieved by the following reaction scheme: 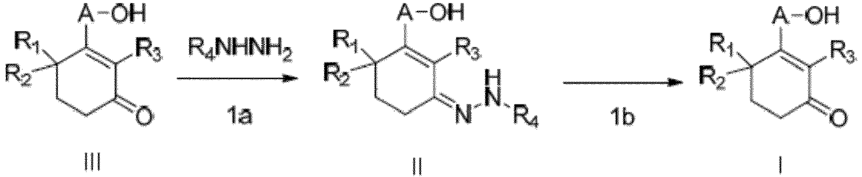 wherein
A represents C₁₀₋₁₈ alkylene;
R₁, R₂, and R₃ each independently represent H or methyl; and
R₄ represents H, substituted or unsubstituted C₁₋₇ alkyl, substituted or unsubstituted C₆₋₁₄ aryl, 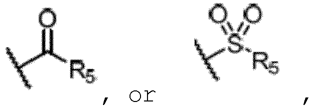
wherein "substituted" means substituted with one substituent or two or more substituents selected from methyl, nitro, chlorine, and bromine; R₅ represents H, methoxy, tert-butoxy, benzyloxy, phenyl, 4-methylphenyl, or amino; and R₄ is preferably 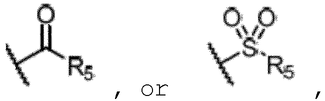
the method comprising the steps of
   (1a) subjecting a cyclohexenone long-chain alcohol crude product III and hydrazine or its derivative R₄NHNH₂ to a condensation reaction to obtain a compound II, and
   (1b) hydrolyzing the compound II in the presence of an acidic substance to obtain the high-purity compound I, wherein the cyclohexenone long-chain alcohol crude product represented by formula III is obtained by the following reaction scheme.

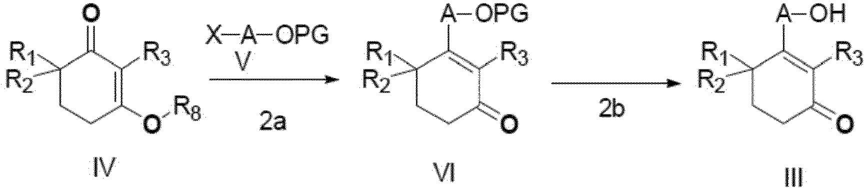

This method comprises the following steps:
(2a) subjecting a compound IV and a compound V to a metal-mediated Barbier reaction to generate a compound VI, and
(2b) subjecting the compound VI to a deprotection reaction in the presence of an acidic substance to directly remove a protective group thereby obtaining the cyclohexenone long-chain alcohol crude product III,
   wherein X represents halogen, R₈ represents C₁₋₇ alkyl, C₆₋₁₄ aryl, or 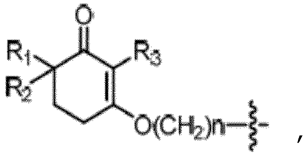 R₁, R₂, R₃, and A are as defined above, n represents 1 to 12, PG represents 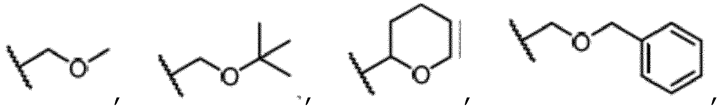 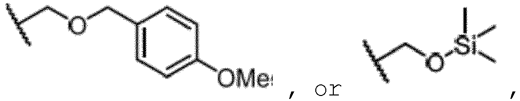 and PG is preferably

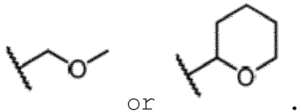

In this method, the metal in step (2a) is lithium, sodium, strontium, magnesium, or zinc, and preferably lithium, strontium, or magnesium; and the molar ratio of the metal to the compound IV is 1:1 to 12:1, and preferably 2:1 to 10:1.

The molar ratio of the compound V to the compound IV is 0.6:1 to 6:1, and preferably 0.8:1 to 4:1.

The Barbier reaction may be performed in the presence or absence of a catalyst, and the catalyst is one member or two or more members selected from tetramethylethylenediamine and hexamethylphosphoric triamide, and the molar ratio of the catalyst to the compound IV is 0.2:1 to 2:1, and preferably 0.4:1 to 1.2:1.

The Barbier reaction is performed in a suitable solvent, and the solvent is one member or two or more members selected from benzene, toluene, chlorobenzene, xylene, tetrahydrofuran, methyltetrahydrofuran, dioxane, methyl tert-butyl ether, n-hexane, n-heptane, cyclohexane, acetonitrile, hexamethylphosphoric triamide, and sulfolane, and preferably one member or two or more members of toluene, xylene, tetrahydrofuran, methyltetrahydrofuran, and n-hexane.

The temperature of the Barbier reaction is selected from -20 to 100°C, preferably -10 to 50°C, and the reaction time is 1 to 36 hours, and preferably 2 to 24 hours.

In this method, the acidic substance in step (2b) is one member or two or more members of methanesulfonic acid, benzenesulfonic acid, p-toluenesulfonic acid, camphorsulfonic acid, pyridinium p-toluenesulfonate, triethylamine hydrochloride, hydrochloric acid, sulfuric acid, phosphoric acid, sodium hydrogensulfate, magnesium hydrogensulfate, an acidic molecular sieve, acidic resin, acetic acid, trifluoroacetic acid, trifluoromethanesulfonic acid, iron trichloride, boron trifluoride ethyl ether, tri silyl chlorosilane, and acetyl chloride, and preferably one member or two or more members of benzenesulfonic acid, p-toluenesulfonic acid, camphorsulfonic acid, pyridinium p-toluenesulfonate, hydrochloric acid, and acetic acid. The molar ratio of the acidic substance to the compound VI is 0.02:1 to 1:1, and preferably 0.05:1 to 0.2:1. The deprotection reaction is performed in a suitable solvent, and the solvent is one member or two or more members of methanol, ethanol, isopropanol, n-butanol, tert-butanol, tert-pentanol, acetonitrile, tetrahydrofuran, methyl tert-butyl ether, isopropyl ether, dioxane, acetone, 2-butanone, ethyl acetate, isobutyl acetate, toluene, xylene, chlorobenzene, benzene, N,N-dimethylacetamide, N,N-dimethylformamide, N,N-diethylformamide, N-methylpyrrolidone, dichloromethane, 1,2-dichloroethane, chloroform, n-hexane, n-heptane, cyclohexane, and water, and preferably one member or two or more members of methanol, ethanol, tetrahydrofuran, acetonitrile, n-heptane, and water.

The temperature of the deprotection reaction is selected from -20 to 100°C, and preferably 0 to 50°C. The reaction time is 0.1 to 10 hours, and preferably 0.5 to 5 hours.

Step (2a) and step (2b) may be separately performed stepwise, or may be performed in a one-pot reaction method.

The present invention further provides a method for producing a high-purity cyclohexenone long-chain alcohol represented by formula I, achieved by the following reaction scheme: 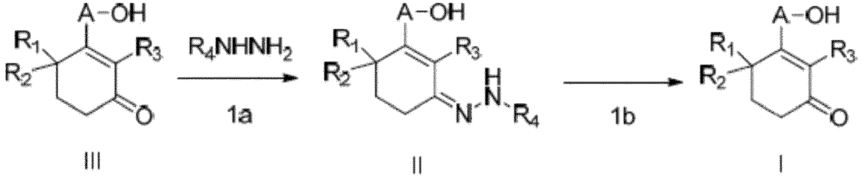 wherein
A represents C₁₀₋₁₈ alkylene;
R₁, R₂, and R₃ each independently represent H or methyl; and
R₄ represents H, substituted or unsubstituted C₁₋₇ alkyl, substituted or unsubstituted C₆₋₁₄ aryl, 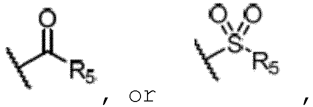
wherein "substituted" means substituted with one substituent or two or more substituents selected from methyl, nitro, chlorine, and bromine; R₅ represents H, methoxy, tert-butoxy, benzyloxy, phenyl, 4-methylphenyl, or amino; and R₄ is preferably 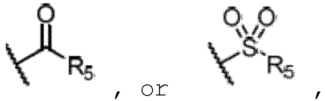
the method comprising the steps of
   (1a) subjecting a cyclohexenone long-chain alcohol crude product III and hydrazine or its derivative R₄NHNH₂ to a condensation reaction to obtain a compound II, and
   (1b) hydrolyzing the compound II in the presence of an acidic substance to obtain the high-purity compound I, wherein the cyclohexenone long-chain alcohol crude product represented by formula III is obtained by the reaction scheme shown below. Specifically, a compound IX is subjected to a metal-mediated intermolecular Barbier reaction, thereby obtaining the cyclohexenone long-chain alcohol crude product III. The reaction is as shown in the following reaction scheme: 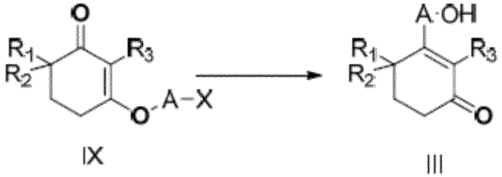 wherein R₁, R₂, and R₃ are as defined above, A represents C₁₀₋₁₈ alkylene, and X represents halogen.

In this method, the metal is lithium, sodium, strontium, magnesium, or zinc, and preferably lithium, strontium, or magnesium.

The molar ratio of the metal to the compound IX is 1:1 to 12:1, and preferably 2:1 to 10:1.

The Barbier reaction may be performed in the presence or absence of a catalyst, and the catalyst is one member or two or more members selected from tetramethylethylenediamine and hexamethylphosphoric triamide. The molar ratio of the catalyst to the compound IX is 0.2 to 2:1, and preferably 0.4 to 1.2:1.

The Barbier reaction is performed in a suitable solvent, and the solvent is one member or two or more members selected from benzene, toluene, chlorobenzene, xylene, tetrahydrofuran, methyltetrahydrofuran, dioxane, methyl tert-butyl ether, n-hexane, n-heptane, cyclohexane, acetonitrile, hexamethylphosphoric triamide, and sulfolane, and preferably one member or two or more members of toluene, xylene, tetrahydrofuran, methyltetrahydrofuran, and n-hexane.

The temperature of the Barbier reaction is selected from -20 to 100°C, and preferably -10 to 50°C. The reaction time is 1 to 36 hours, and preferably 2 to 24 hours.

### Advantageous Effect

The present invention provides a method for producing and purifying a high-purity cyclohexenone long-chain alcohol, and achieves the production of a cyclohexenone long-chain alcohol by a one-pot method using a metal-mediated Barbier reaction, instead of the Grignard reaction (which requires the separate production of a Grignard reagent alone) disclosed in the literature. The product is purified by a condensation reaction with hydrazine or its derivative, thereby avoiding the operation of column chromatography.

The method of the present invention is performed in a short scheme, is easy to handle and perform, easy to control, provides a high-purity product at a high yield, and is a simple, highly efficient, economical, and industrial production method.

### Description of Embodiments

The following describes the present invention in more detail with reference to Examples. However, the following embodiments are simply described as examples of the present invention, and these Examples are not intended to limit the present invention in any manner. It is clear that a person skilled in the art can make various replacements or modifications within the scope of the present invention as defined in the claims. The present invention should be construed as being intended to cover replacements and modifications made within the scope of the claims attached to this specification.

### Preparation of Compound IV

### Preparation Example 1: 3-isobutoxy-2,6,6-trimethylcyclohex-2-en-1-one

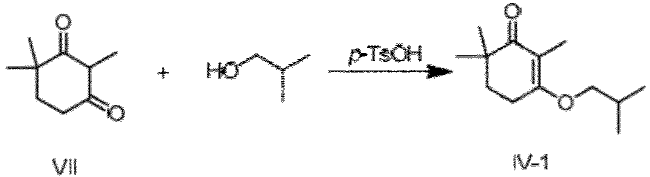

2,4,4-trimethylcyclohexane-1,3-dione VII (80 g, 1 eq) and isobutanol (76.9 g, 2 eq) were added to cyclohexane (400 mL), and p-TSA·H₂O (5 g, 0.05 eq) was added thereto, followed by heating under reflux for 16 hours to separate water. The reaction mixture was subjected to a post-treatment and cooled to ambient temperature. The resultant was then sequentially washed with 5% sodium hydroxide (80 mL), water (80 mL), and a saturated sodium chloride solution (80 mL), and dried over anhydrous sodium sulfate, followed by concentration by drying, thereby obtaining 3-isobutoxy-2,6,6-trimethylcyclohex-2-en-1-one (103.65 g, 95%). ¹H NMR (400 MHz, CDCl3): δ 3.77 (d, 2 H, J = 6.4 Hz), 2.55-2.58 (m, 2 H), 1.95-2.05 (m, 1 H), 1.82 (t, 2 H, J = 6.4 Hz), 1.72 (s, 3 H), 1.11 (s, 6 H), 1.01 (d, 6 H, J = 6.4 Hz).

### Preparation Example 2: 3-cyclohexylmethoxy-2,6,6-trimethylcyclohex-2-en-1-one

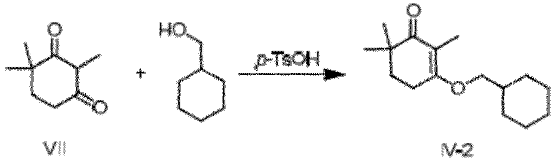

2,4,4-trimethylcyclohexane-1,3-dione VII (10 g, 1 eq) and cyclohexyl methanol (14.8 g, 2 eq) were added to cyclohexane (100 mL), and p-TSA·H₂O (0.62 g, 0.05 eq) was added thereto, followed by heating under reflux for 16 hours to separate water. The reaction mixture was subjected to a post-treatment and cooled to ambient temperature. The resultant was then sequentially washed with 5% sodium hydroxide (20 mL), water (20 mL), and a saturated sodium chloride solution (20 mL), and dried over anhydrous sodium sulfate, followed by concentration by drying, and purification by column chromatography, thereby obtaining 3-cyclohexylmethoxy-2,6,6-trimethylcyclohex-2-en-1-one (14.8 g, 91%) .
¹H NMR (400 MHz, CDCl₃) δ 3.80 (d, J = 6.9 Hz, 1 H), 2.55 (td, 1 H, J = 6.2, 1.1 Hz), 1.83 (m, 6 H), 1.75 (m, 3 H), 1.72 (s, 3 H), 1.36-1.23 (m, 6 H), 1.12 (s, 6 H).

### Preparation Example 3: 3,3'-(ethyl-1,2-dioxy)-di(2,6,6-trimethylcyclohex-2-en-1-one)

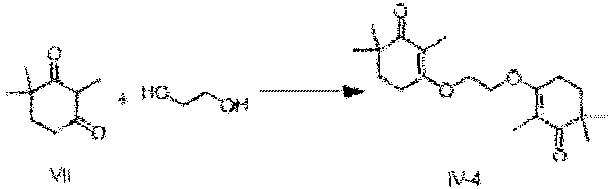

2,4,4-trimethylcyclohexane-1,3-dione VII (5 g, 1 eq), ethylene glycol (1.01 g, 0.5 eq), p-TSA·H₂O (311 mg, 0.05 eq), and toluene (30 mL) were added to a flask, and heated under reflux for 6 hours to separate water. The toluene was dried by rotation, and a saturated sodium hydrogen carbonate solution and dichloromethane were added, followed by extraction. The dichloromethane layer was further washed with a saturated sodium chloride solution once, dried over anhydrous sodium sulfate, and dried by rotation. A mixture solvent of petroleum ether and ethyl acetate was added thereto, and the solids were precipitated, followed by stirring for 3 hours, and further followed by suction filtration and drying, thereby obtaining 3,3'-(ethyl-1,2-dioxy)-di(2,6,6-trimethylcyclohex-2-en-1-one)(4.3 g, 80%). The melting point was 131 to 132°C.
¹H NMR (300 MHz, CDCl₃) δ 4.25 (s, 4 H), 2.57 (t, 4 H, J = 6.2 Hz), 1.81 (t, 4 H, J = 6.3 Hz), 1.68 (s, 6 H), 1.07 (s, 12 H).

### Preparation Example 4: 3-methoxy-2,6,6-trimethylcyclohex-2-en-1-one

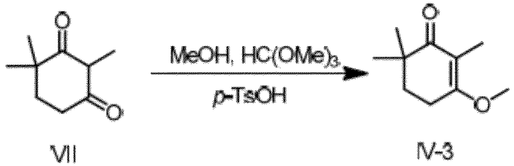

2,4,4-trimethylcyclohexane-1,3-dione VII (2.7 g, 1 eq) and trimethyl orthoformate (2.8 g, 1.5 eq) were added to methanol (40 mL), and p-TSA·H₂O (167 mg, 0.05 eq) was added thereto, followed by stirring at room temperature overnight. The reaction mixture was subjected to a post-treatment, and dichloromethane (30 mL) was added for dilution. The resultant was washed sequentially with 5% sodium hydroxide (20 mL), water (10 mL), and a saturated sodium chloride solution (10 mL), and dried over anhydrous sodium sulfate, followed by concentration by drying and purification by column chromatography, thereby obtaining 3-methoxy-2,6,6-trimethylcyclohex-2-en-1-one (2.19 g, 74.4%).
¹H NMR (400 MHz, CDCl₃): δ 3.81 (s, 3 H), 2.55-2.58 (m, 2 H), 1.95-2.05 (m, 1 H), 1.82 (t, 2 H, J = 6.4 Hz), 1.72 (s, 3 H), 1.11 (s, 6 H) .

### Preparation Example 5: 3,3'-(propyl-1,2-dioxy)-di(2,6,6-trimethylcyclohex-2-en-1-one)

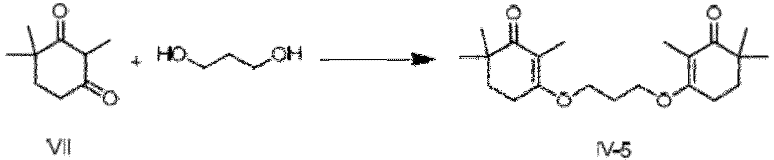

2,4,4-trimethylcyclohexane-1,3-dione VII (5 g, 1 eq), 1,3-propane diol (1.23 g, 0.5 eq), p-TSA·H₂O (311 mg, 0.05 eq), and toluene (30 mL) were added to a flask and heated under reflux for 6 hours to separate water. The toluene was dried by rotation, and a saturated sodium hydrogen carbonate aqueous solution and dichloromethane were added, followed by extraction. The dichloromethane layer was further washed with a saturated sodium chloride solution once, dried over anhydrous sodium sulfate, and dried by rotation, followed by column chromatography, thereby obtaining 3,3'-(propyl-1,2-dioxy)-di(2,6,6-trimethylcyclohex-2-en-1-one) (3.96 g, 70%).
¹H NMR (400 MHz, CDCl₃) δ 4.13 (m, 4 H), 2.46 (t, 4 H, J = 6.2 Hz), 1.81 (t, J = 6.2 Hz, 4 H), 1.70 (s, 6 H), 1.32 (t, 2 H, J = 6.2 Hz), 1.08 (s, 12 H).

### Preparation Example 6: 3,3'-(butyl-1,2-dioxy)-di(2,6,6-trimethylcyclohex-2-en-1-one)

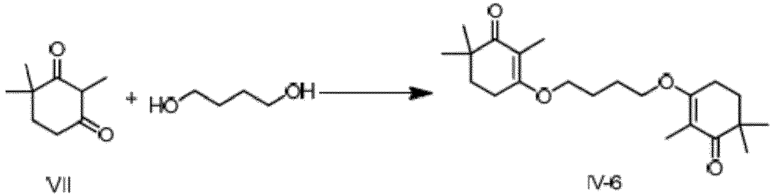

2,4,4-trimethylcyclohexane-1,3-dione VII (5 g, 1 eq) was dissolved in toluene, and p-TSA·H₂O (280 mg, 0.05 eq) and 1,4-butanediol (1.46 g, 0.5 eq) were added thereto, followed by heating under reflux to separate water. The reaction mixture was cooled to room temperature, and a saturated sodium carbonate solution was added, followed by the addition of ethyl acetate for extraction. The organic layer was washed with a saturated sodium chloride solution, dried over anhydrous sodium sulfate, concentrated, and triturated with a petroleum ether ethyl acetate mixture solvent, followed by filtration, thereby obtaining a compound 3,3'-(butyl-1,2-dioxy)-di(2,6,6-trimethylcyclohex-2-en-1-one) (4.3 g, 74%). The melting point was 132 to 134°C.
¹H NMR (300 MHz, CDCl₃) δ 4.10 (m, 4 H), 2.57 (t, 4 H, J = 6.2 Hz), 1.83 (m, 4 H), 1.78 (t, 4 H, J = 6.2 Hz) 1.70 (s, 6 H), 1.08 (s, 12 H) .

### Preparation Example 7: 3,3'-(pentyl-1,2-dioxy)-di(2,6,6-trimethylcyclohex-2-en-1-one)

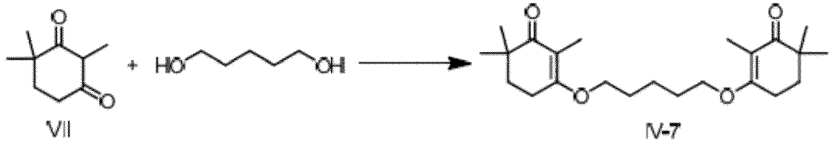

2,4,4-trimethylcyclohexane-1,3-dione VII (5 g, 1 eq) was dissolved in toluene, and p-TSA·H₂O (280 mg, 0.05 eq) and 1,4-pentane diol (1.69 g, 0.5 eq) were added thereto, followed by heating under reflux to separate water. The reaction mixture was cooled to room temperature, and a saturated sodium carbonate solution was added, followed by the addition of ethyl acetate for extraction. The organic layer was washed with a saturated sodium chloride solution, dried over anhydrous sodium sulfate, and concentrated, followed by column chromatography, thereby obtaining a compound 3,3'-(pentyl-1,2-dioxy)-di(2,6,6-trimethylcyclohex-2-en-1-one) (3.66 g, 60%).
¹H NMR (300 MHz, CDCl₃) δ 4.10 (m, 4 H), 2.57 (t, 4 H, J = 6.2 Hz), 1.83 (m, 6H), 1.78 (t, 4H, J = 6.2 Hz) 1.70 (s, 6H), 1.08 (s, 12 H) .

### Preparation Example 8: 3,3'-(hexyl-1,2-dioxy)-di(2,6,6-trimethylcyclohex-2-en-1-one)

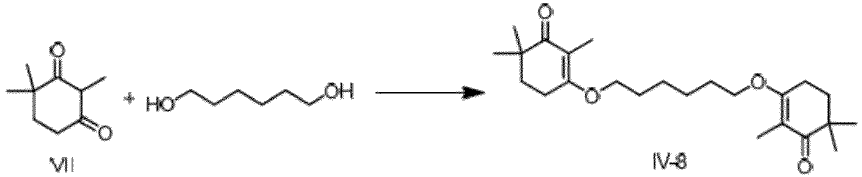

2,4,4-trimethylcyclohexane-1,3-dione VII (5 g, 1 eq) and 1,6-hexane diol (1.92 g, 0.5 eq) were dissolved in toluene (50 mL), and camphorsulfonic acid (1.5 g, 0.2 eq) was added thereto, followed by heating under reflux overnight to separate water. The reaction mixture was cooled to ambient temperature, and washed individually with 5% sodium hydroxide (20 mL), water (10 mL), and a saturated sodium chloride solution (20 mL). The resultant was dried over anhydrous sodium sulfate, filtered, and dried by concentration, followed by trituration with methanol, thereby obtaining 3,3'-(hexyl-1,2-dioxy)-di(2,6,6-trimethylcyclohex-2-en-1-one)(4.7 g, 89%) as white solids. The melting point was 92 to 94°C.
¹H NMR (300 MHz, CDCl₃) δ 4.10 (m, 4 H), 2.47 (t, 4 H, J = 6.2 Hz), 1.88 (m, 4H), 1.78 (t, 4H, J = 6.2 Hz) 1.70 (s, 6H), 1.32 (m, 4 H) 1.08 (s, 12 H).

### Preparation of Compound IX

### Preparation Example 9: 3-(15-chloropentadecyloxy-2,6,6-trimethylcyclohex-2-en-1-one)

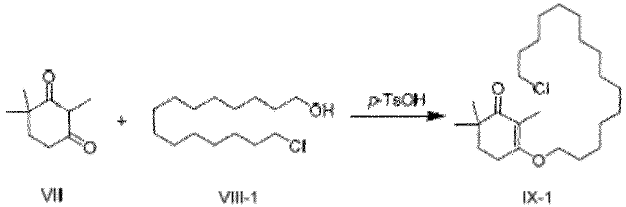

2,4,4-trimethylcyclohexane-1,3-dione VII (1.3 g, 1.1 eq) and 15-chloropentadecanol VIII-1 (2 g, 1 eq) were added to cyclohexane (50 mL), and p-TSA·H₂O (70 mg, 0.05 eq) was added thereto, followed by heating under reflux for 16 hours to separate water. The reaction mixture was subjected to a post-treatment, cooled to ambient temperature, and sequentially washed with 5% sodium hydroxide (20 mL), water (10 mL), and a saturated sodium chloride solution (10 mL). The resultant was dried over anhydrous sodium sulfate and dried by concentration, thereby obtaining 3-(15-chloropentadecyloxy)-2,6,6-trimethylcyclohex-2-en-1-one (2.46 g, 80.9%).
¹H NMR (400 MHz, CDCl₃): δ 3.97 (t, 2 H, J = 6.8 Hz), 3.45 (m, 2 H, J = 6.8Hz), 2.54-2.55 (m, 2 H), 1.78-1.84 (m, 4 H), 1.68 (s, 3 H), 1.39-1.41 (m, 4 H), 1.22-1.35 (m, 21 H), 1.08 (s, 6 H).

### Preparation Example 10: 3-(15-bromopentadecyloxy)-2,6,6-trimethylcyclohex-2-en-1-one

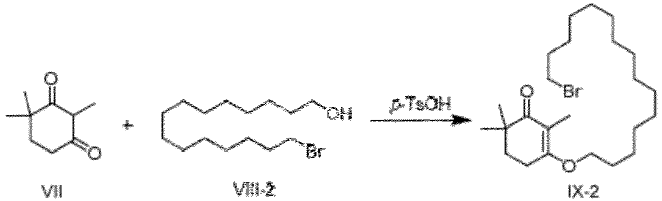

2,4,4-trimethylcyclohexane-1,3-dione VII (1.5 g, 1 eq) and 15-bromopentadecanol VIII-2 (2.5 g, 1 eq) were added to cyclohexane (50 mL), and p-TSA·H₂O (80 mg, 0.05 eq) was added thereto, followed by heating under reflux for 16 hours to separate water. The reaction mixture was subjected to a post-treatment, cooled to ambient temperature, and sequentially washed with 5% sodium hydroxide (20 mL), water (10 mL), and a saturated sodium chloride solution (10 mL). The resultant was dried over anhydrous sodium sulfate and dried by concentration, thereby obtaining 3-(15-bromopentadecyloxy)-2,6,6-trimethylcyclohex-2-en-1-one (3.37 g, 93.6%).
¹H NMR (400 MHz, CDCl₃) : δ 3.97 (t, 2 H, J = 6.8Hz), 3.40 (m, 2 H, J = 6.8Hz), 2.54-2.55 (m, 2 H), 1.78-1.84 (m, 4 H), 1.68 (s, 3 H), 1.39-1.41 (m, 4 H), 1.22-1.35 (m, 21 H), 1.08 (s, 6 H).

### Preparation Example 11: 3-(15-iodopentadecyloxy)-2,6,6-trimethylcyclohex-2-en-1-one

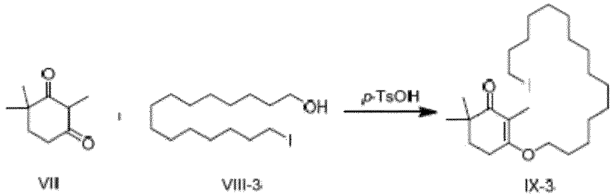

2,4,4-trimethylcyclohexane-1,3-dione VII (2.55 g, 1.2 eq) and 15-iodopentadecanol VIII-3 (5 g, 1 eq) were added to cyclohexane (50 mL), and p-TSA·H₂O (130 mg, 0.05 eq) was added thereto, followed by heating under reflux for 16 hours to separate water. The reaction mixture was subjected to a post-treatment, cooled to ambient temperature, sequentially washed with 5% sodium hydroxide (20 mL), water (10 mL), and a saturated sodium chloride solution (10 mL). The resultant was dried over anhydrous sodium sulfate and dried by concentration, thereby obtaining 3-(15-iodopentadecyloxy)-2,6,6-trimethylcyclohex-2-en-1-one (5.3 g, 76.6%).
¹H NMR (400 MHz, CDCl₃): δ 3.97 (t, 2 H, J = 8.4 Hz), 3.18 (m, 2 H, J = 9.6 Hz), 2.54 (t, 2 H, J = 8.4 Hz), 1.78-1.83 (m, 2 H), 1.68 (s, 3 H), 1.21-1.50 (m, 25 H), 1.08 (s, 6 H).

### Preparation of Compound VI

### Example 1: 2,4,4-trimethyl-3-[15-(tetrahydro-2-hydro-pyranyl)-2-oxy-pentadecyl]cyclohex-2-en-1-one

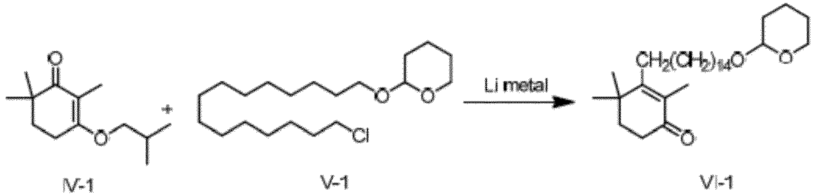

3-isobutoxy-2,6,6-trimethylcyclohex-2-en-1-one IV-1 (3 g, 1 eq) and 2-(15-chloropentadecyl)oxytetrahydro-2-hydro-pyran V-1 (5.44 g, 1.1 eq) were added to a three-necked flask, and replacement by nitrogen gas was performed three times. Tetrahydrofuran or toluene was added thereto, and replacement by nitrogen gas was performed three times. Li (297 g, 3 eq) was added thereto, and replacement by nitrogen gas was performed three times. The temperature was controlled to 25 to 30°C, and the reaction was allowed to proceed for 16 hours. TLC confirmed that the starting materials almost completely reacted. The reaction mixture was cooled to 10 to 20°C, and saturated ammonium chloride (30 mL) was added dropwise, followed by the addition of water (30 mL). The mixture was stirred for 5 minutes and separated into layers. The organic layer was washed with 0.5M hydrochloric acid (20 mL), and allowed to stand to separate into layers. The resultant was washed with a saturated sodium chloride solution and dried over anhydrous sodium sulfate, followed by concentration by drying, thereby obtaining 2,4,4-trimethyl-3-[15-(tetrahydro-2-hydro-pyranyl)-2-oxy-pentadecyl]cyclohex-2-en-1-one (crude product: 6.6 g, 103%) as a lime green oil.
¹H NMR (400 MHz, CDCl₃) δ 4.59 (dd, 1 H, J = 4.5, 2.7 Hz), 3.89 (ddd, 1 H, J = 11.1, 7.4, 3.4 Hz), 3.75 (dt, 1 H, J = 9.5, 6.9 Hz), 3.59-3.47 (m, 1H), 3.40 (dt, 1 H, J = 9.6, 6.7 Hz), 2.51-2.43 (m, 2 H), 2.23-2.14 (m, 2 H), 1.89-1.80 (m, 4 H), 1.77 (s, 3 H), 1.67-1.48 (m, 6 H), 1.49-1.28 (m, 23 H), 1.17 (s, 6 H), 1.14 (d, 1 H, J = 14.2 Hz).

### Example 2: 2,4,4-trimethyl-3-[15-(tetrahydro-2-hydro-pyranyl)-2-oxy-pentadecyl]cyclohex-2-en-1-one

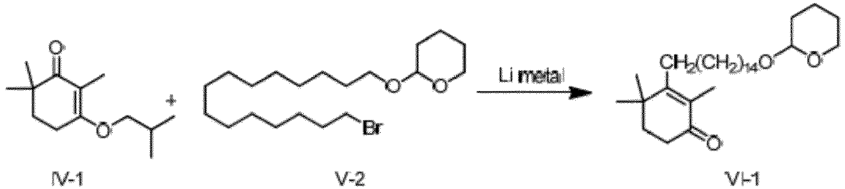

3-isobutoxy-2,6,6-trimethylcyclohex-2-en-1-one (5 g, 1 eq) and 2-(15-bromopentadecyl)oxytetrahydro-2-hydro-pyran (12.1 g, 1.3 eq) were dissolved in THF and toluene, and replacement by nitrogen gas was performed. Li (500 mg, 3 eq) was added thereto, and the mixture was stirred at 15 to 25°C overnight. The next day, TLC confirmed that the starting materials completely reacted. The reaction mixture was cooled to about 20°C, and a saturated ammonium chloride solution (20 mL) was added dropwise to the reaction mixture, followed by supplementation with water (20 mL) and stirring to separate the mixture into layers. The organic layer was washed with 0.5N hydrochloric acid (20 mL), and washed with water, followed by drying and concentration by drying, thereby obtaining 2,4,4-trimethyl-3-[15-(tetrahydro-2-hydro-pyranyl)-2-oxy-pentadecyl]cyclohex-2-en-1-one (crude product: 13.5 g, 126%) as an oil.

### Example 3: 2,4,4-trimethyl-3-[15-(tetrahydro-2-hydro-pyranyl)-2-oxy-pentadecyl]cyclohex-2-en-1-one

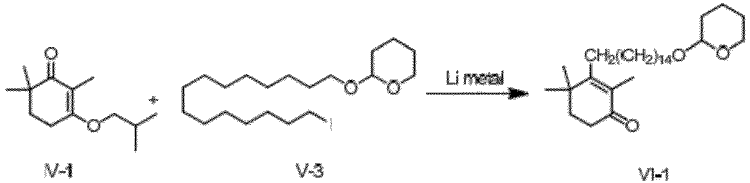

3-isobutoxy-2,6,6-trimethylcyclohex-2-en-1-one IV-1 (5 g, 1 eq) and 2-(15-iodopentadecyl)oxytetrahydro-2-hydro-pyran V-3 (13.6 g, 1.3 eq) were dissolved in THF and toluene, and replacement by nitrogen gas was performed. Li (500 mg, 3 eq) was added thereto, and the mixture was stirred at 15 to 25°C overnight. The next day, TLC confirmed that the starting materials completely reacted. The reaction mixture was cooled to about 20°C, and a saturated ammonium chloride solution (20 mL) was added dropwise to the reaction mixture, followed by supplementation with water (20 mL) and stirring to separate the mixture into layers. The organic layer was washed with 0.5N hydrochloric acid (20 mL) and washed with water, followed by drying, and concentration by drying, thereby obtaining 2,4,4-trimethyl-3-[15-(tetrahydro-2-hydro-pyranyl)-2-oxy-pentadecyl]cyclohex-2-en-1-one (13.5 g, 126%) as an oil.

### Example 4: 3-(15-methoxymethyleneoxy-pentadecyl)-2,4,4-trimethylcyclohex-2-en-1-one

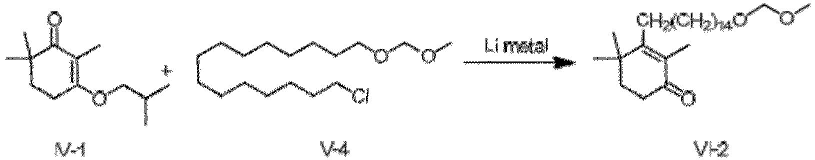

3-isobutoxy-2,6,6-trimethylcyclohex-2-en-1-one IV-1 (3 g, 1 eq) and 1-chloro-15-methoxymethyleneoxypentadecane V-4 (4.82 g, 1.1 eq) were added to a three-necked flask, and replacement by nitrogen gas was performed three times. Tetrahydrofuran and toluene were added thereto, and replacement by nitrogen gas was performed three times. Li (297 g, 3 eq) was added thereto, and replacement by nitrogen gas was performed three times. The temperature was controlled to 25 to 30°C, and a reaction was allowed to proceed for 16 hours. TLC confirmed that the starting materials almost completely reacted. The reaction mixture was cooled to 10 to 20°C, and a saturated ammonium chloride solution (30 mL) was added dropwise thereto, followed by the addition of water (30 mL) and stirring for 5 minutes to separate the mixture into layers. The organic layer was washed with 0.5M hydrochloric acid (20 mL) and allowed to stand to separate into layers. The resultant was washed with a saturated sodium chloride solution, dried over anhydrous sodium sulfate, and dried by concentration, thereby obtaining 3-(15-methoxymethyleneoxy-pentadecyl)-2,4,4-trimethylcyclohex-2-en-1-one (crude product: 6.8 g, 117%) as a lime green oil.
¹H NMR (400 MHz, CDCl₃) δ 4.55 (s, 2 H), 3.49 (t, 2 H, J = 7.4 Hz), 3.16 (s, 3 H), 2.92 (t, 2 H, J = 5.9 Hz), 2.30- 2.22 (m, 2 H), 1.99 (s, 2 H), 1.63-1.68 (m, 2 H), 1.52-1.42 (m, 2 H), 1.42- 1.31 (m, 3 H), 1.34-1.25 (m, 22 H), 1.21 (s, 6H).

### Example 5: 2,4,4-trimethyl-3-[15-(tetrahydro-2-hydro-pyranyl)-2-oxy-pentadecyl]cyclohex-2-en-1-one

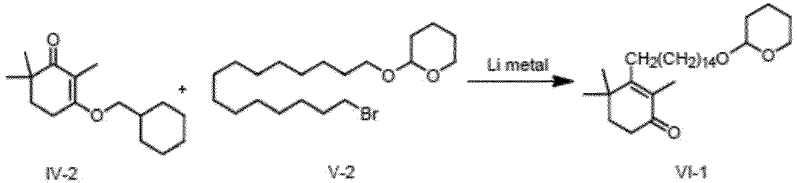

3-cyclohexylmethoxy-2,6,6-trimethylcyclohex-2-en-1-one IV-2 (2 g, 1 eq) and 2-(15-bromopentadecyl)oxytetrahydro-2-hydropyran V-2 (3.44 g, 1.1 eq) were dissolved in THF (30 mL), and replacement by nitrogen gas was performed. Li (166 mg, 3 eq) was added thereto, and the mixture was stirred at 20 to 30°C overnight. The next day, TLC confirmed that the starting materials completely reacted. A saturated ammonium chloride solution (10 mL) and water (10 mL) were added dropwise to the reaction mixture, and the mixture was stirred for 10 minutes, followed by separation into layers. EA was added to the organic layer for dilution, and the resultant was washed with water, washed with a saturated sodium chloride solution, and dried, followed by concentration by drying, thereby obtaining 2,4,4-trimethyl-3-[15-(tetrahydro-2-hydro-pyranyl)-2-oxy-pentadecyl]cyclohex-2-en-1-one (crude product: 4.6 g, 128%) as an oil.

### Example 6: 2,4,4-trimethyl-3-[15-(tetrahydro-2-hydro-pyranyl)-2-oxy-pentadecyl]cyclohex-2-en-1-one

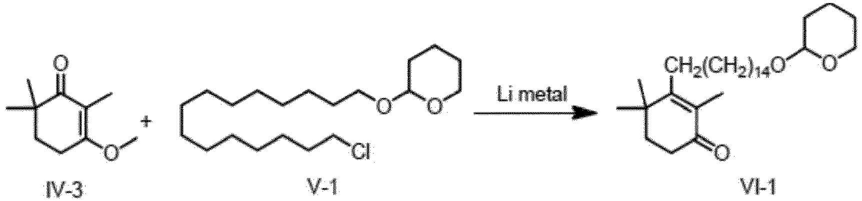

3-isomethoxy-2,6,6-trimethylcyclohex-2-en-1-one IV-3 (10 g, 1 eq) and 2-(15-chloropentadecyl)oxytetrahydro-2-hydropyran V-1 (22.7 g, 1.1 eq) were added to a three-necked flask, and replacement by nitrogen gas was performed three times. Tetrahydrofuran or toluene was added thereto, and replacement by nitrogen gas was performed three times. Li (1.24 g, 3 eq) was added thereto, and replacement by nitrogen gas was performed three times. The temperature was controlled to 25 to 30°C, and a reaction was allowed to proceed for 16 hours. TLC confirmed that the starting materials almost completely reacted. The reaction mixture was cooled to 10 to 20°C, and a saturated ammonium chloride solution (100 mL) was added dropwise thereto, followed by the addition of water (100 mL) and stirring for 5 minutes to separate the mixture into layers. The organic layer was washed with 0.5M hydrochloric acid (60 mL), and allowed to stand to separate into layers. The resultant was washed with a saturated sodium chloride solution, dried over anhydrous sodium sulfate, and dried by concentration, thereby obtaining 2,4,4-trimethyl-3-[15-(tetrahydro-2-hydro-pyranyl)-2-oxy-pentadecyl]cyclohex-2-en-1-one (crude product: 28.2 g, 106%) as a lime green oil.

### Preparation of Compound III

### Example 7: 3-(15-hydroxypentadecyl)-2,4,4-trimethylcyclohex-2-en-1-one

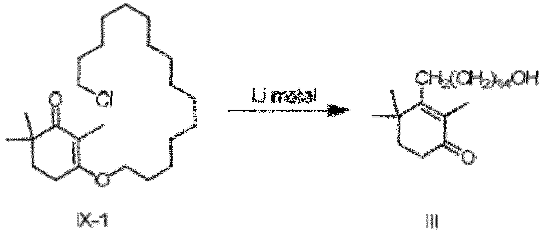

3-(15-chloropentadecyloxy-2,6,6-trimethylcyclohex-2-en-1-one) IX-1 (2 g) was added to anhydrous tetrahydrofuran (20 mL), and protected by nitrogen gas. Lithium (104 mg, 3 eq) was added thereto, and replacement by nitrogen gas was performed, followed by stirring at room temperature for more than 16 hours. TLC confirmed that the starting materials completely reacted. A saturated ammonium chloride solution (10 mL) and water (10 mL) were added dropwise to the reaction mixture, and the mixture was stirred for 10 minutes to separate the mixture into layers. EA was added to the organic layer for dilution, and the resultant was washed with water, washed with a saturated sodium chloride solution, and dried, thereby obtaining a 3-(15-hydroxypentadecyl)-2,4,4-trimethylcyclohex-2-en-1-one crude product as an oil (1.82 g, 100%). The content (HPLC external standard method) was 61.2%.

### Example 8: 3-(15-hydroxypentadecyl)-2,4,4-trimethylcyclohex-2-en-1-one

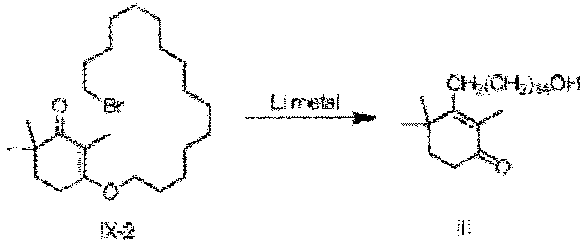

3-(15-bromopentadecyloxy)-2,6,6-trimethylcyclohex-2-en-1-one IX-2 (2 g) was added to anhydrous tetrahydrofuran (20 mL), and protected by nitrogen gas. Lithium (94 mg, 3 eq) was added thereto, and replacement by nitrogen gas was performed, followed by stirring at room temperature for more than 16 hours. TLC confirmed that the starting materials completely reacted. A saturated ammonium chloride solution (10 mL) and water (10 mL) were added dropwise to the reaction mixture, and the mixture was stirred for 10 minutes to separate the mixture into layers. EA was added to the organic layer for dilution, and the resultant was washed with water, washed with a saturated sodium chloride solution, and dried, thereby obtaining a 3-(15-hydroxypentadecyl)-2,4,4-trimethylcyclohex-2-en-1-one crude product as an oil (1.67 g, 102%). The content (HPLC external standard method) was 63.3%.

### Example 9: 3-(15-hydroxypentadecyl)-2,4,4-trimethylcyclohex-2-en-1-one

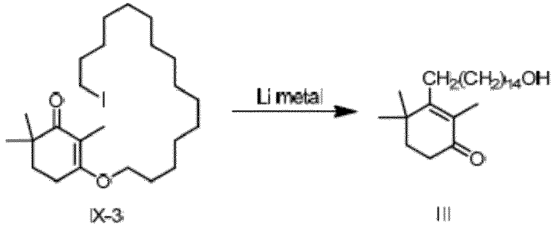

3-(15-iodopentadecyloxy)-2,6,6-trimethylcyclohex-2-en-1-one IX-3 (2 g) was added to anhydrous tetrahydrofuran (20 mL), and protected by nitrogen gas. Lithium (85 mg, 3 eq) was added thereto, and replacement by nitrogen gas was performed, followed by stirring at room temperature for more than 16 hours. TLC confirmed that the starting materials completely reacted. A saturated ammonium chloride (10 mL) and water (10 mL) were added dropwise to the reaction mixture, and the mixture was stirred for 10 minutes to separate the mixture into layers. EA was added to the organic layer for dilution, and the resultant was washed with water, washed with a saturated sodium chloride solution, and dried, thereby obtaining a 3-(15-hydroxypentadecyl)-2,4,4-trimethylcyclohex-2-en-1-one crude product as an oil (1.50 g, 101%). The content (HPLC external standard method) was 60.5%.

### Preparation of Compound VI

### Example 10: 2,4,4-trimethyl-3-[15-(tetrahydro-2-hydro-pyranyl)-2-oxy-pentadecyl]cyclohex-2-en-1-one

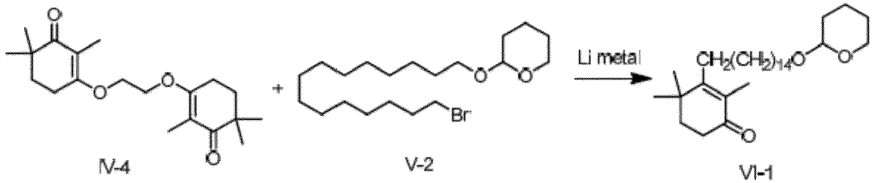

3,3'-(ethyl-1,2-dioxy)-di(2,6,6-trimethylcyclohex-2-en-1-one) IV-3 (5 g, 1 eq) and 2-(15-bromopentadecyl)oxytetrahydro-2-hydro-pyran (12.8 g, 2.2 eq) were dissolved in THF (50 mL), and replacement by nitrogen gas was performed. Li (623 mg, 6 eq) was added thereto, and stirred at 25 to 35°C overnight. The next day, TLC confirmed the end of the reaction. The reaction mixture was cooled to 0 to 10°C, and a saturated ammonium chloride (20 mL) and water (10 mL) were added dropwise, followed by separation into layers. The organic layer was washed with 0.5N hydrochloric acid (20 mL), washed with a saturated sodium chloride solution, and dried over anhydrous sodium sulfate, followed by filtration and concentration by drying, thereby obtaining a 2,4,4-trimethyl-3-[15-(tetrahydro-2-hydro-pyranyl)-2-oxy-pentadecyl]cyclohex-2-en-1-one crude product (13.9 g, 104%).

### Example 11: 2,4,4-trimethyl-3-[15-(tetrahydro-2-hydro-pyranyl)-2-oxy-pentadecyl]cyclohex-2-en-1-one

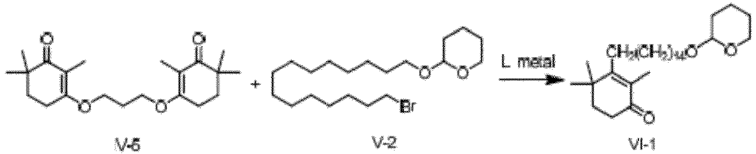

3,3'-(propyl-1,2-dioxy)-di(2,6,6-trimethylcyclohex-2-en-1-one) (5 g, 1 eq) and 2-(15-bromopentadecyl)oxytetrahydro-2-hydro-pyran (12.4 g, 2.2 eq) were dissolved in THF (50 mL), and replacement by nitrogen gas was performed. Li (597 mg, 6 eq) was added thereto, and the mixture was stirred at 25 to 35°C overnight. The next day, TLC confirmed the end of the reaction. The reaction mixture was cooled to 0 to 10°C, and a saturated ammonium chloride (20 mL) and water (10 mL) were added dropwise to separate the mixture into layers. The organic layer was washed with 0.5N hydrochloric acid (20 mL), washed with a saturated sodium chloride solution, dried over anhydrous sodium sulfate, and filtered, followed by concentration by drying, thereby obtaining 2,4,4-trimethyl-3-[15-(tetrahydro-2-hydro-pyranyl)-2-oxy-pentadecyl]cyclohex-2-en-1-one (crude product: 7.05 g, 109%).

### Example 12: 2,4,4-trimethyl-3-[15-(tetrahydro-2-hydro-pyranyl)-2-oxy-pentadecyl]cyclohex-2-en-1-one

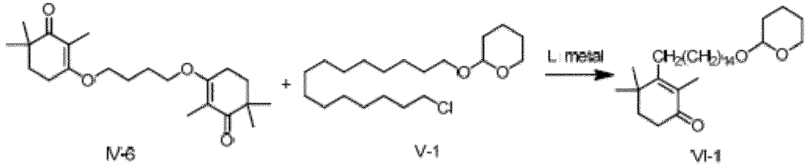

3,3'-(butyl-1,2-dioxy)-di(2,6,6-trimethylcyclohex-2-en-1-one) IV-6 (5 g, 1 eq) and 2-(15-chloropentadecyl)oxytetrahydro-2-hydro-pyran V-1 (10.5 g, 2.2 eq) were dissolved in THF (50 mL), and replacement by nitrogen gas was performed. Li (574 mg, 6 eq) was added thereto, and the mixture was stirred at 25 to 35°C overnight. The next day, TLC confirmed the end of the reaction, and the reaction mixture was cooled to 0 to 10°C. A saturated ammonium chloride (20 mL) and water (10 mL) were added dropwise thereto to separate the mixture into layers. The organic layer was washed with 0.5N hydrochloric acid (20 mL), washed with a saturated sodium chloride solution, dried over anhydrous sodium sulfate, and filtered, followed by concentration by drying, thereby obtaining 2,4,4-trimethyl-3-[15-(tetrahydro-2-hydro-pyranyl)-2-oxy-pentadecyl]cyclohex-2-en-1-one (crude product: 7.1 g, 115%).

### Example 13: 3-(15-methoxymethyleneoxy-pentadecyl)-2,4,4-trimethyl-cyclohex-2-en-1-one

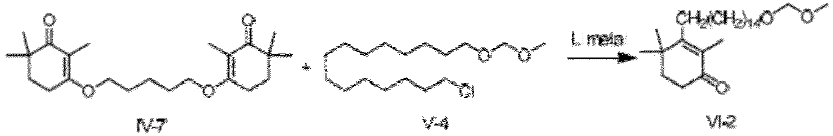

3,3'-(pentyl-1,1-dioxy)-di(2,6,6-trimethylcyclohex-2-en-1-one) IV-7 (5 g, 1 eq) and 1-chloro-15-methoxymethyleneoxypentadecane V-4 (8.97 g, 2.2 eq) were dissolved in THF (50 mL), and replacement by nitrogen gas was performed. Li (553 mg, 6 eq) was added thereto, and the mixture was stirred at 25 to 35°C overnight. The next day, TLC confirmed the end of the reaction, and the reaction mixture was cooled to 0 to 10°C. Saturated ammonium chloride (20 mL) and water (10 mL) were added dropwise to separate the mixture into layers. The organic layer was washed with 0.5N hydrochloric acid (20 mL), washed with a saturated sodium chloride solution, dried over anhydrous sodium sulfate, and filtered, followed by concentration by drying, thereby obtaining 3-(15-methoxymethyleneoxy-pentadecyl)-2,4,4-trimethylcyclohex-2-en-1-one (crude product: 5.5 g, 101%).

### Example 14: 2,4,4-trimethyl-3-[15-(tetrahydro-2-hydro-pyranyl)-2-oxy-pentadecyl]cyclohex-2-en-1-one

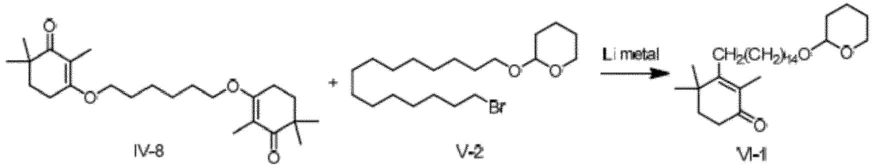

3,3'-(hexyl-1,2-dioxy)-di(2,6,6-trimethylcyclohex-2-en-1-one) IV-8 (5 g, 1 eq) and 2-(15-bromopentadecyl)oxytetrahydro-2-hydro-pyran (12.1 g, 2.2 eq) were dissolved in THF (50 mL), and replacement by nitrogen gas was performed. Li (530 mg, 6 eq) was added thereto, and the mixture was stirred at 25 to 35°C overnight. The next day, TLC confirmed the end of the reaction, and the reaction mixture was cooled to 0 to 10°C. Saturated ammonium chloride (20 mL) and water (10 mL) were added dropwise to separate the mixture into layers. The organic layer was washed with 0.5N hydrochloric acid (20 mL), washed with a saturated sodium chloride solution, dried over anhydrous sodium sulfate, and filtered, followed by concentration by drying, thereby obtaining a 2,4,4-trimethyl-3-[15-(tetrahydro-2-hydro-pyranyl)-2-oxy-pentadecyl]cyclohex-2-en-1-one crude product (13.6 g, 118%).

### Preparation of Compound III

### Example 15: 3-(15-hydroxypentadecyl)-2,4,4-trimethylcyclohex-2-en-1-one

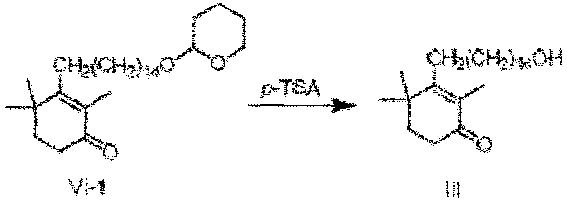

2,4,4-trimethyl-3-[15-(tetrahydro-2-hydro-pyranyl)-2-oxy-pentadecyl]cyclohex-2-en-1-one VI-1 (51.7 g, 1 eq) was dissolved in methanol (200 mL), and p-TSA·H₂O (1.8 g, 0.1 eq) was added thereto, followed by stirring for 3 hours. Sodium hydrogen carbonate (2 g) was added, and the mixture was stirred for 10 minutes, and dried by concentration. Dichloromethane (100 mL) and water (50 mL) were added thereto to separate the mixture into phases. The organic layer was washed with a saturated sodium chloride solution (50 mL), dried over anhydrous sodium sulfate, and dried by concentration, thereby obtaining 3-(15-hydroxypentadecyl)-2,4,4-trimethylcyclohex-2-en-1-one (crude product: 39.8 g). The content (HPLC external standard method) was 70.2%.

### Example 16: 3-(15-hydroxypentadecyl)-2,4,4-trimethylcyclohex-2-en-1-one

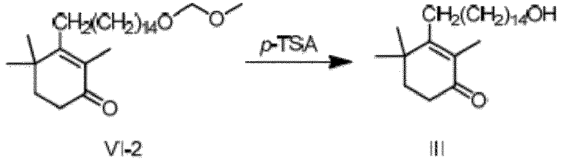

3-(15-methoxymethyleneoxy-pentadecyl)-2,4,4-trimethylcyclohex-2-en-1-one VI-2 (50 g, 1 eq) was dissolved in methanol (200 mL), and p-TSA·H₂O (2.11 g, 0.1 eq) was added thereto, followed by stirring for 3 hours. Sodium hydrogen carbonate (2 g) was added, and the mixture was stirred for 10 minutes, and dried by concentration. Dichloromethane (100 mL) and water (50 mL) were added to separate the mixture into layers. The organic layer was washed with a saturated sodium chloride solution (50 mL), dried over anhydrous sodium sulfate, and dried by concentration, thereby obtaining 3-(15-hydroxypentadecyl)-2,4,4-trimethylcyclohex-2-en-1-one (crude product: 42.8 g). The content (HPLC external standard method) was 69.6%.

### Example 17: 3-(15-hydroxypentadecyl)-2,4,4-trimethylcyclohex-2-en-1-one (one-pot method)

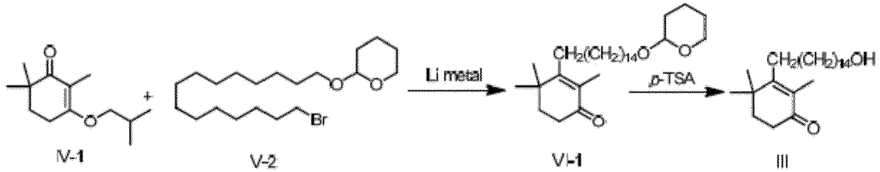

3-isobutoxy-2,6,6-trimethylcyclohex-2-en-1-one (50 g, 1 eq) and 2-(15-bromopentadecyl)oxytetrahydro-2-hydro-pyran V-2 (121 g, 1.3 eq) were dissolved in THF, and replacement by nitrogen gas was performed. Li (5 g, 3 eq) was added thereto, and the mixture was stirred at 15 to 25°C overnight. The next day, TLC confirmed that the starting materials completely reacted. The reaction mixture was cooled to about 20°C, and a saturated ammonium chloride solution (200 mL) was added dropwise to the reaction mixture, followed by supplementation with water (200 mL) and stirring to separate the mixture into layers. The organic layer was washed with 0.5N hydrochloric acid (200 mL) and washed with water. Methanol (400 mL) and p-TSA·H₂O (4.7 g, 0.1 eq) were added thereto, and the mixture was stirred for 3 hours. Sodium hydrogen carbonate (5.22 g) was added, and the mixture was stirred for 10 minutes, followed by concentration by drying. Dichloromethane (200 mL) and water (100 mL) were added thereto to separate the mixture into layers. The organic layer was washed with a saturated sodium chloride solution (100 mL), dried over anhydrous sodium sulfate, and dried by concentration, thereby obtaining a 3-(15-hydroxypentadecyl)-2,4,4-trimethylcyclohex-2-en-1-one crude product (103.9 g, 120%). The content (HPLC external standard method) was 68.5%.

### Preparation of Compound II

### Example 18: [3-(15-hydroxypentadecyl)-2,4,4-trimethylcyclohex-2-ene]-aminoformylhydrazone

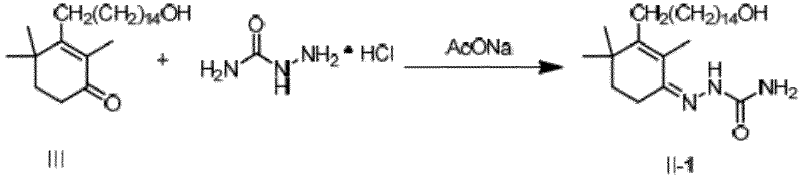

3-(15-hydroxypentadecyl)-2,4,4-trimethylcyclohex-2-en-1-one crude product III (obtained in Example 17, content: 68.5%) (10.4 g, 1 eq) was dissolved in ethanol (70 mL), and water (35 mL) was added thereto. Semicarbazide hydrochloride (3.98 g, 1.5 eq) was added, and anhydrous sodium acetate (3.9 g, 2 eq) was added, followed by stirring to completely dissolve them. The solution was then heated under reflux overnight with stirring to precipitate solids. TLC confirmed that the starting materials completely reacted. The solvent was removed by concentration, and water (50 mL) was added thereto, followed by trituration at room temperature for 30 minutes. The resultant was filtered, and washed with water, followed by trituration the solids with acetonitrile (50 mL) for 30 minutes. The resultant was then filtered and dried by heating, thereby obtaining 8.14 g of 3-(15-hydroxypentadecyl)-2,4,4-trimethylcyclohex-2-ene-1-aminoformylhydrazone as whitish solids (calculated based on compound IV-1, three-step yield: 81.2%). The melting point was 150 to 152°C.
¹H NMR (400 MHz, DMSO-d₆) δ 8.98 (s, 1 H), 6.24 (s, 2 H), 4.32 (s, 1 H), 3.35 (d, 5 H, J = 13.8 Hz), 2.35 (d, 1 H, J = 7.3 Hz), 2.11 (s, 2 H), 1.79 (s, 2 H), 1.51 (s, 1 H), 1.39 (s, 2 H), 1.33 (s, 8 H), 1.25 (s, 16 H), 1.02 (s, 6 H).

| Wavelength | Target Compound II-1 Purity | Impurities Content | | |
|---|---|---|---|---|
| | | Hydrazone impurities resulting from non-desorption of hydroxy-protecting groups | Starting Materials | Other Individual Impurities |
| 210 nm | 98.99% | 0.79% | 0.09% | Less than 0.15% |
| 254 nm | 99.07% | 0.68% | 0.17% | Less than 0.15% |

### Example 19: [3-(15-hydroxypentadecyl)-2,4,4-trimethylcyclohex-2-ene]-4-methylbenzolsulfonyl hydrazone

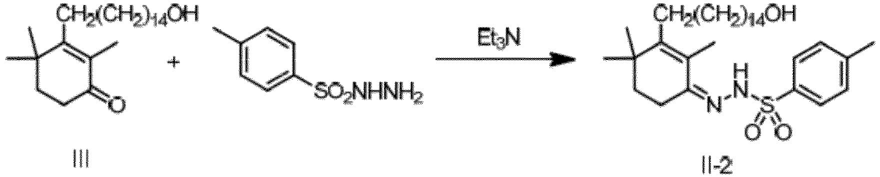

3-(15-hydroxypentadecyl)-2,4,4-trimethylcyclohex-2-en-1-one crude product III (5 g, 1 eq) (obtained in Example 15, content: 70.2%) was dissolved in ethanol (50 mL), and water (25 mL) was added thereto. p-Toluenesulfonyl hydrazone (6.38 g, 2.5 eq) was added, and triethylamine (2.78 g, 2 eq) was added, followed by stirring to completely dissolve them. The solution was heated until the internal temperature reached 60 to 70°C and stirred overnight to precipitate solids. TLC confirmed that the starting materials completely reacted. The solvent was removed by concentration, and water (50 mL) was added thereto, followed by trituration at room temperature for 30 minutes. The resultant was filtered, and washed with water, followed by triturating the solids with acetonitrile (50 mL) for 30 minutes. The resultant was filtered and dried by heating, thereby obtaining 3.56 g of 3-(15-hydroxypentadecyl)-2,4,4-trimethylcyclohex-2-ene-1-aminoformylhydrazone as whitish solids (calculated based on compound IV-1, three-step yield: 81.2%).
¹H NMR (400 MHz, CDCl₃) δ 7.82-7.80 (m, 2 H), 7.33-7.31 (m, 2 H), 4.30 (t, 2 H, J = 7.0 Hz), 2.42 (s, 2 H), 2.30-2.22 (m, 2 H), 2.11 (t, J = 7.1 Hz, 2 H), 1.89 (s, 2 H), 1.49 (dt, J = 14.1, 7.0 Hz, 4 H), 1.42-1.22 (m, 25 H), 1.02 (s, 6H).

| Wavelength | Target Compound II-2 Purity | Impurities Content | | |
|---|---|---|---|---|
| | | Hydrazone impurities resulting from non-desorption of hydroxy-protecting groups | Starting Materials | Other Individual Impurities |
| 210 nm | 98.43% | 1.34% | 0.08% | Less than 0.15% |
| 254 nm | 98.64% | 1.05% | 0.21% | Less than 0.15% |

### Preparation of Compound I

### Example 20: 3-(15-hydroxypentadecyl)-2,4,4-trimethylcyclohex-2-en-1-one

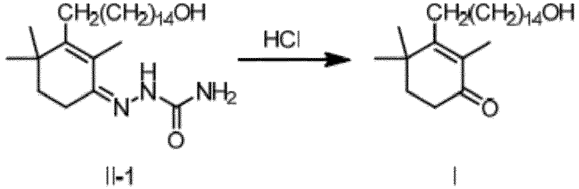

[3-(15-hydroxypentadecyl)-2,4,4-trimethylcyclohex-2-ene]-aminoformylhydrazone II-1 (100 g) was added to THF (200 mL) and 3N hydrochloric acid (500 mL), and protected by nitrogen gas. The solution was heated to 55 to 60°C and stirred for 2 hours to separate it into layers to form two phases, followed by cooling to 45°C, thereby separating it into layers. The aqueous layer was separated and removed, and the organic layer was dispersed in n-heptane (200 mL), followed by sequential washing with a saturated sodium hydrogen carbonate solution (200 mL) and a saturated sodium chloride solution (100 mL). The resultant was dried over anhydrous sodium sulfate, and activated carbon (5 g) was added thereto, followed by stirring for 20 minutes. The mixture was filtered and dried by concentration. n-heptane (1000 mL) was added to dissolve it, and the temperature was decreased to 0 to 10°C with stirring, followed by stirring for 2 hours. The resultant was filtered, and the solids were dried under reduced pressure, thereby obtaining 3-(15-hydroxypentadecyl)-2,4,4-trimethylcyclohex-2-en-1-one (79 g, 91%) as whitish solids or a pale-color oil. The melting point was 36 to 38°C.
¹H NMR (400 MHz, CDCl₃): δ 3.61 (t, 2 H, J = 6.8 Hz), 2.43 (t, 2 H, J = 9.6 Hz), 2.13-2.17 (m, 2 H), 1.77-1.80 (m, 3 H), 1.73 (s, 3 H), 1.49-1.55 (m, 2 H), 1.21-1.42 (m, 24 H), 1.13 (s, 6 H). HPLC: 99.98%, individual impurities < 0.05% (210 nm, 254 nm).

### Example 21: 3-(15-hydroxypentadecyl)-2,4,4-trimethylcyclohex-2-en-1-one

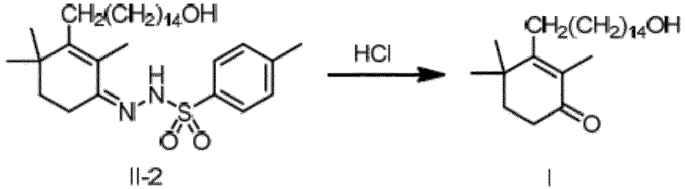

[3-(15-hydroxypentadecyl)-2,4,4-trimethylcyclohexyl-2-ene]-methylbenzolsulfonyl hydrazone II-2 (50 g) was added to THF (100 mL) and 3N hydrochloric acid (250 mL), and protected by nitrogen gas. The solution was heated to 55 to 60°C and stirred for 2 hours to separate it into layers to form two phases, followed by cooling to 45°C, thereby separating it into layers. The aqueous layer was separated and removed, and the organic layer was dispersed in n-heptane (100 mL), followed by sequential washing with saturated sodium hydrogen carbonate (100 mL) and a saturated sodium chloride solution (50 mL). The resultant was dried over anhydrous sodium sulfate, and activated carbon (5 g) was added thereto, followed by stirring for 20 minutes. The mixture was filtered and dried by concentration. n-heptane (1000 mL) was added to dissolve it, and the temperature was decreased to 0 to 10°C with stirring, followed by stirring for 2 hours. The resultant was filtered, and the solids were dried under reduced pressure, thereby obtaining, 3-(15-hydroxypentadecyl)-2,4,4-trimethylcyclohex-2-en-1-one (27.4 g, 80%) as whitish solids or a pale-color oil.
¹H NMR (400 MHz, CDCl₃): δ 3.61 (t, 2 H, J = 6.8 Hz), 2.43 (t, 2 H, J = 9.6 Hz), 2.13-2.17 (m, 2 H), 1.77-1.80 (m, 3 H), 1.73 (s, 3 H), 1.49-1.55 (m, 2 H), 1.21-1.42 (m, 24 H), 1.13 (s, 6 H). HPLC: 99.95%, individual impurities < 0.05% (210 nm, 254 nm).

## Claims

1. A method for producing a high-purity cyclohexenone long-chain alcohol represented by formula I, achieved by the following reaction scheme: 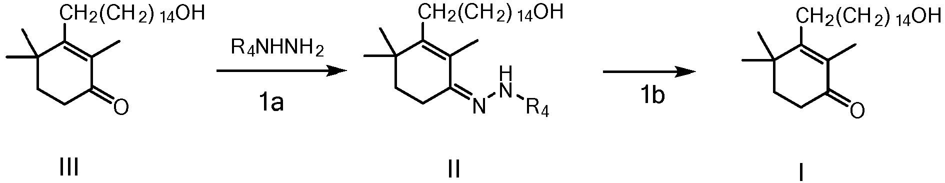 wherein
R₄ represents 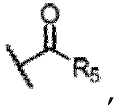
R₅ represents amino,
the method comprising the steps of
(1a) subjecting a cyclohexenone long-chain alcohol crude product III and hydrazine derivative R₄NHNH₂ to a condensation reaction to obtain a compound II, and
(1b) hydrolyzing the compound II in the presence of an acidic substance to obtain the high-purity compound I.

2. The production method according to claim 1, wherein the high-purity cyclohexenone long-chain alcohol represented by formula I has a purity by HPLC of more than 95%; preferably, the high-purity cyclohexenone long-chain alcohol represented by formula I has a purity by HPLC of more than 99%; and more preferably, the high-purity cyclohexenone long-chain alcohol represented by formula I has a purity by HPLC of more than 99.9%.

3. The production method according to claim 1, wherein
step (1a) is performed in the presence of an acid, an alkali, or a desiccant;
the alkali is one member or two or more members selected from sodium alkoxide, potassium alkoxide, magnesium oxide, calcium oxide, sodium carbonate, potassium carbonate, lithium carbonate, cesium carbonate, calcium carbonate, sodium acetate, potassium acetate, lithium acetate, sodium benzoate, potassium benzoate, lithium benzoate, triethylamine, trimethylamine, diisopropylethylamine, 1,8-diazabicyclo[5.4.0]undec-7-ene, 1,5-diazabicyclo[4.3.0]non-5-ene, and triethylenediamine, and preferably one member or two or more members selected from sodium tert-butoxide, potassium tert-butoxide, sodium carbonate, potassium carbonate, sodium acetate, potassium acetate, triethylamine, and diisopropylethylamine;
the acid is one member or two or more members selected from acetic acid, benzoic acid, hydrochloric acid, sulfuric acid, phosphoric acid, benzenesulfonic acid, p-toluenesulfonic acid, camphorsulfonic acid, boron trifluoride ethyl ether, scandium trifluoromethanesulfonate, indium trifluoromethanesulfonate, and bismuth trifluoromethanesulfonate, and preferably one member or two or more members selected from acetic acid, p-toluenesulfonic acid, boron trifluoride ethyl ether, and bismuth trifluoromethanesulfonate; and
the desiccant is one member or two or more members selected from desiccants, such as a molecular sieve, magnesium sulfate, sodium sulfate, and calcium hydride, and preferably one member or two or more members selected from a molecular sieve and magnesium sulfate.

4. The production method according to claim 1, wherein the molar ratio of the hydrazine or its derivative R₄NHNH₂ to the cyclohexenone long-chain alcohol crude product III is 0.8:1 to 3:1, and preferably 0.9:1 to 2:1.

5. The production method according to claim 1, wherein
the condensation reaction is performed in a solvent;
the solvent is one member or two or more members selected from methanol, ethanol, isopropanol, n-butanol, tert-butanol, tert-pentanol, acetonitrile, tetrahydrofuran, methyl tert-butyl ether, isopropyl ether, dioxane, acetone, 2-butanone, ethyl acetate, isobutyl acetate, toluene, xylene, chlorobenzene, benzene, N,N-dimethylacetamide, N,N-dimethylformamide, N,N-diethylformamide, N-methylpyrrolidone, dichloromethane, 1,2-dichloroethane, chloroform, n-hexane, n-heptane, cyclohexane, and water, and preferably one member or two or more members selected from methanol, ethanol, tetrahydrofuran, acetonitrile, and n-heptane;
the temperature of the condensation reaction is 0 to 149°C, and preferably 20 to 129°C; and
the reaction time of the condensation reaction is 0.5 to 24 hours, and preferably 1 to 10 hours.

6. The production method according to claim 1, wherein
the acidic substance recited in step (1b) is one member or two or more members of an organic acid, an inorganic acid, a Lewis acid, an acid salt, and other acidic substance;
the inorganic acid is sulfuric acid, hydrochloric acid, phosphoric acid, polyphosphoric acid, or phosphotungstic acid;
the organic acid is formic acid, acetic acid, propionic acid, oxalic acid, fumaric acid, maleic acid, trifluoroacetic acid, methanesulfonic acid, benzenesulfonic acid, p-toluenesulfonic acid, camphorsulfonic acid, or trifluoromethanesulfonic acid;
the Lewis acid is boron trifluoride ethyl ether, aluminum trichloride, iron trichloride, bismuth trifluoromethanesulfonate, or scandium trifluoromethanesulfonate;
the acid salt is an acid salt, such as sodium hydrogensulfate, ammonium hydrogensulfate, magnesium hydrogensulfate, pyridinium p-toluenesulfonate, triethylamine hydrochloride, and pyridine hydrochloride;
the other acidic substance is silica gel or acidic resin;
preferably the acidic substance is p-toluenesulfonic acid, hydrochloric acid, sulfuric acid, sodium hydrogensulfate, or magnesium hydrogensulfate; and
the molar ratio of the added amount of the compound II to the added amount of the acidic substance is 1:0.2 to 1:10, and preferably 1:0.2 to 1:2.

7. The production method according to claim 1, wherein
the hydrolysis reaction is performed in a solvent;
the solvent is one member or two or more members selected from benzene, toluene, chlorobenzene, xylene, acetonitrile, 2-butanone, acetone, 1,2-dimethyl-2-imidazolone, dimethyl sulfoxide, dimethyl sulfone, sulfolane, hexamethylphosphoric triamide, N,N-dimethylformamide, N,N-dimethylacetamide, N,N-diethylformamide, N-methylpyrrolidone, methanol, ethanol, isopropanol, n-butanol, ethylene glycol, polyethylene glycol, dioxane, methyl tert-butyl ether, isopropyl ether, tetrahydrofuran, n-hexane, cyclohexane, dichloromethane, 1,2-dichloroethane, chloroform, and water, and preferably the solvent is one member or two or more members selected from toluene, acetonitrile, methanol, ethanol, water, tetrahydrofuran, methyl tert-butyl ether, and dichloromethane;
the reaction temperature of the hydrolysis reaction is 20 to 139°C, and the reaction time of the hydrolysis reaction is 0.5 to 24 hours, and
preferably the reaction temperature of the hydrolysis reaction is 20 to 100°C, and the reaction time of the hydrolysis reaction is 0.5 to 10 hours.

8. A method for producing a high-purity cyclohexenone long-chain alcohol represented by formula I, achieved by the following reaction scheme: 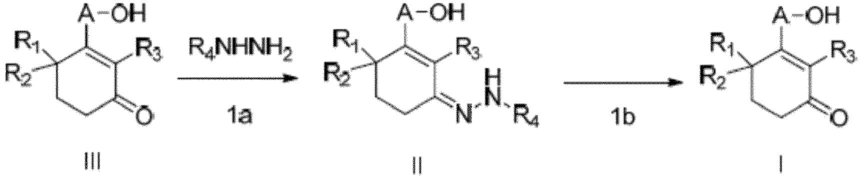 wherein
A represents C₁₀₋₁₈ alkylene;
R₁, R₂, and R₃ each independently represent H or methyl; and
R₄ represents H, substituted or unsubstituted C₁₋₇ alkyl, substituted or unsubstituted C₆₋₁₄ aryl, 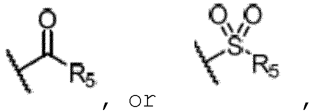
wherein "substituted" means substituted with one substituent or two or more substituents selected from methyl, nitro, chlorine, and bromine; R₅ represents H, methoxy, tert-butoxy, benzyloxy, phenyl, 4-methylphenyl, or amino; and R₄ is preferably 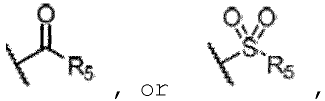
the method comprising the steps of
(1a) subjecting a cyclohexenone long-chain alcohol crude product III and hydrazine or its derivative R₄NHNH₂ to a condensation reaction to obtain a compound II, and
(1b) hydrolyzing the compound II in the presence of an acidic substance to obtain the high-purity compound I, wherein the cyclohexenone long-chain alcohol crude product III is obtained by the following reaction scheme: 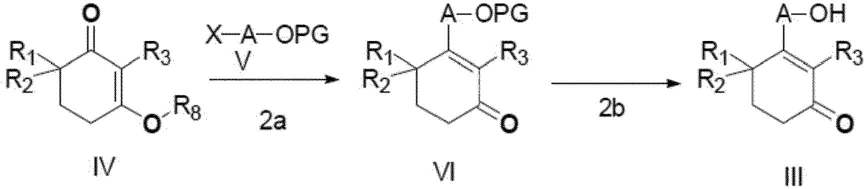
wherein X represents halogen, R₈ represents C₁₋₇ alkyl, C₆₋₁₄ aryl, or 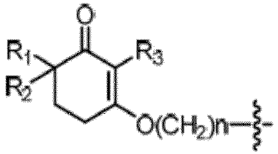 wherein n represents 1 to 12,
PG represents 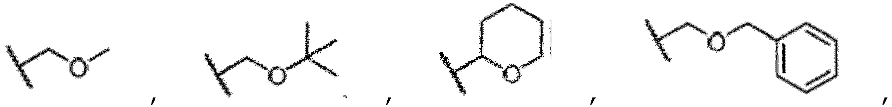 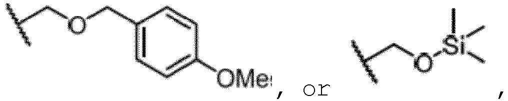
and PG is preferably 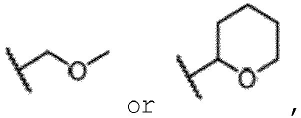
the method comprising the steps of
(2a) subjecting a compound IV and a compound V to a metal-mediated Barbier reaction to generate a compound VI, and
(2b) subjecting the compound VI to a deprotection reaction in the presence of an acidic substance to remove a protective group, thereby obtaining the cyclohexenone long-chain alcohol crude product III.

9. The production method according to claim 8, wherein in step (2a),
the metal is lithium, sodium, strontium, magnesium, or zinc, and preferably lithium, strontium, or magnesium,
the molar ratio of the metal to the compound IV is 1:1 to 12:1, and preferably 2:1 to 10:1, and
the molar ratio of the compound V to the compound IV is 0.6:1 to 6:1, and preferably 0.8:1 to 4:1.

10. The production method according to claim 8, wherein
the Barbier reaction is performed in the presence or absence of a catalyst,
the catalyst is one member or two or more members selected from tetramethylethylenediamine and hexamethylphosphoric triamide,
the molar ratio of the catalyst to the compound IV is 0.2:1 to 2:1, and preferably 0.4:1 to 1.2:1,
the Barbier reaction is performed in a suitable solvent,
the solvent is one member or two or more members selected from benzene, toluene, chlorobenzene, xylene, tetrahydrofuran, methyltetrahydrofuran, dioxane, methyl tert-butyl ether, n-hexane, n-heptane, cyclohexane, acetonitrile, hexamethylphosphoric triamide, and sulfolane, and preferably one member or two or more members of toluene, xylene, tetrahydrofuran, methyltetrahydrofuran, and n-hexane,
the temperature of the Barbier reaction is selected from -20 to 100°C, and preferably -10 to 50°C, and
the reaction time of the Barbier reaction is 1 to 36 hours, and preferably 2 to 24 hours.

11. The production method according to claim 8, wherein in step (2b),
the acidic substance is one member or two or more members of methanesulfonic acid, benzenesulfonic acid, p-toluenesulfonic acid, camphorsulfonic acid, pyridinium p-toluenesulfonate, triethylamine hydrochloride, hydrochloric acid, sulfuric acid, phosphoric acid, sodium hydrogensulfate, magnesium hydrogensulfate, an acidic molecular sieve, acidic resin, acetic acid, trifluoroacetic acid, trifluoromethanesulfonic acid, iron trichloride, boron trifluoride ethyl ether, and acetyl chloride, and preferably one member or two or more members of benzenesulfonic acid, p-toluenesulfonic acid, camphorsulfonic acid, pyridinium p-toluenesulfonate, hydrochloric acid, and acetic acid,
the molar ratio of the acidic substance to the compound VI is 0.02:1 to 1:1, and preferably 0.05:1 to 0.2:1,
the deprotection reaction is performed in a suitable solvent, and the solvent is one member or two or more members of methanol, ethanol, isopropanol, n-butanol, tert-butanol, tert-pentanol, acetonitrile, tetrahydrofuran, methyl tert-butyl ether, isopropyl ether, dioxane, acetone, 2-butanone, ethyl acetate, isobutyl acetate, toluene, xylene, chlorobenzene, benzene, N,N-dimethylacetamide, N,N-dimethylformamide, N,N-diethylformamide, N-methylpyrrolidone, dichloromethane, 1,2-dichloroethane, chloroform, n-hexane, n-heptane, cyclohexane, and water, and preferably one member or two or more members of methanol, ethanol, tetrahydrofuran, acetonitrile, n-heptane, and water,
the reaction temperature of the deprotection reaction is -20 to 100°C, and preferably 0 to 50°C,
the reaction time of the deprotection reaction is 0.1 to 10 hours, and preferably 0.5 to 5 hours.

12. The production method according to claim 8, wherein step (2a) and step (2b) may be separately performed stepwise, or may be performed by a one-pot reaction method.

13. A method for producing a high-purity cyclohexenone long-chain alcohol represented by formula I, achieved by the following reaction scheme: 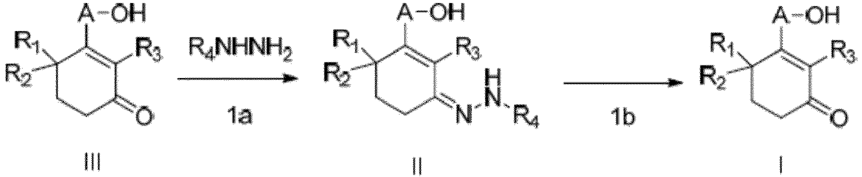 wherein
A represents C₁₀₋₁₈ alkylene;
R₁, R₂, and R₃ each independently represent H or methyl; and
R₄ represents H, substituted or unsubstituted C₁₋₇ alkyl, substituted or unsubstituted C₆₋₁₄ aryl, 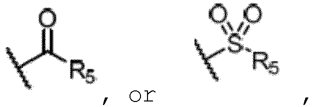
wherein "substituted" means substituted with one substituent or two or more substituents selected from methyl, nitro, chlorine, and bromine; R₅ represents H, methoxy, tert-butoxy, benzyloxy, phenyl, 4-methylphenyl, or amino; and R₄ is preferably 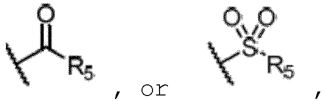
the method comprising the steps of
(1a) subjecting a cyclohexenone long-chain alcohol crude product III and hydrazine or its derivative R₄NHNH₂ to a condensation reaction to obtain a compound II, and
(1b) hydrolyzing the compound II in the presence of an acidic substance to obtain the high-purity compound I, wherein the cyclohexenone long-chain alcohol crude product III is obtained by the following reaction scheme: 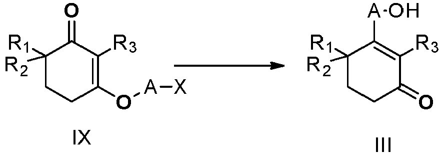
wherein a compound IX is subjected to a metal-mediated intermolecular Barbier reaction to obtain the cyclohexenone long-chain alcohol crude product III, R₁, R₂, and R₃ are as defined above, X represents halogen, and A represents C₁₀₋₁₈ alkylene.

14. The production method according to claim 13, wherein
the metal is lithium, sodium, strontium, magnesium, or zinc, and preferably lithium, strontium, or magnesium, and
the molar ratio of the metal to the compound IX is 1:1 to 12:1, and preferably 2:1 to 10:1.

15. The production method according to claim 13, wherein
the Barbier reaction is performed in the presence or absence of a catalyst,
the catalyst is one member or two or more members selected from tetramethylethylenediamine and hexamethylphosphoric triamide,
the molar ratio of the catalyst to the compound IX is 0.2 to 2:1, and preferably 0.4 to 1.2:1,
the Barbier reaction is performed in a suitable solvent, and the solvent is one member or two or more members selected from benzene, toluene, chlorobenzene, xylene, tetrahydrofuran, methyltetrahydrofuran, dioxane, methyl tert-butyl ether, n-hexane, n-heptane, cyclohexane, acetonitrile, hexamethylphosphoric triamide, and sulfolane, and preferably one member or two or more members of toluene, xylene, tetrahydrofuran, methyltetrahydrofuran, and n-hexane,
the reaction temperature of the Barbier reaction is -20 to 100°C, and preferably -10 to 50°C, and
the reaction time of the Barbier reaction is 1 to 36 hours, and preferably 2 to 24 hours.

16. [3-(15-hydroxypentadecyl)-2,4,4-trimethylcyclohex-2-ene]-aminoformylhydrazone

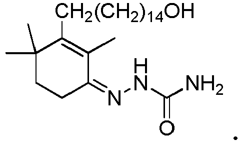

17. A compound represented by formula IX 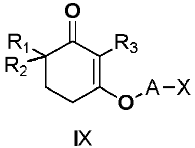 wherein R₁, R₂, and R₃ each independently represent H or methyl, X represents halogen, and A represents C₁₀₋₁₈ alkylene.

18. The compound according to claim 17, which is one selected from 3-(15-chloropentadecyloxy-2,6,6-trimethylcyclohex-2-en-1-one) 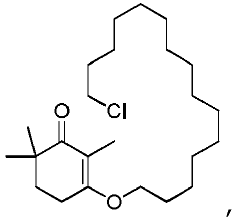 3-(15-bromopentadecyloxy)-2,6,6-trimethylcyclohex-2-en-1-one 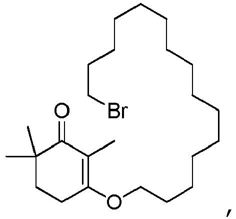 and
3-(15-iodopentadecyloxy)-2,6,6-trimethylcyclohex-2-en-1-one

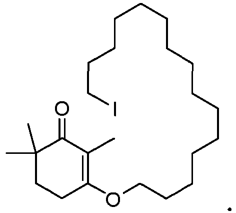

19. Use of the compound according to claim 17 or 18 for producing a compound represented by formula III as defined in claim 1.

20. Use of the compound according to claim 16 for producing a compound represented by formula I as defined in claim 1

21. 3-(15-methoxymethyleneoxy-pentadecyl)-2,4,4-trimethylcyclohex-2-en-1-one

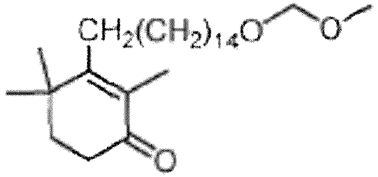

## Patentansprüche

1. Verfahren zur Herstellung eines durch Formel I dargestellten hochreinen langkettigen Cyclohexenon-Alkohols, erzielt durch das folgende Reaktionsschema: 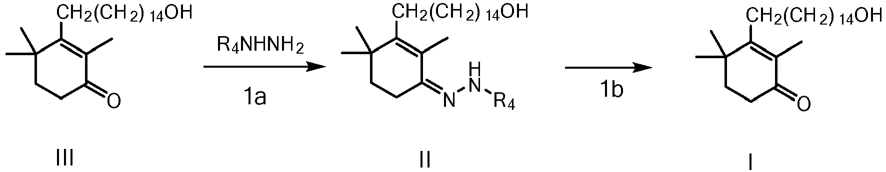 worin
R₄ 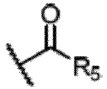 darstellt,
R₅ Amino darstellt,
wobei das Verfahren die Schritte
(1a) Unterziehen eines langkettigen Cyclohexenon-Alkohol-Rohprodukts III und einem Hydrazinderivat R₄NHNH₂ einer Kondensationsreaktion, um eine Verbindung II zu erhalten, und
(1b) Hydrolysieren der Verbindung II in Gegenwart einer sauren Substanz, um die hochreine Verbindung I zu erhalten,
umfasst.

2. Herstellungsverfahren gemäß Anspruch 1, worin der durch Formel I dargestellte hochreine langkettige Cyclohexenon-Alkohol eine HPLC-Reinheit von mehr als 95 % aufweist, der durch Formel I dargestellte hochreine langkettige Cyclohexenon-Alkohol vorzugsweise eine HPLC-Reinheit von mehr als 99 % aufweist und der durch Formel I dargestellte hochreine langkettige Cyclohexenon-Alkohol besonders bevorzugt eine HPLC-Reinheit von mehr als 99,9 % aufweist.

3. Herstellungsverfahren gemäß Anspruch 1, worin
Schritt (1a) in Gegenwart einer Säure, eines Alkalis oder eines Trockenmittels durchgeführt wird,
das Alkali mindestens ein Vertreter oder zwei oder mehr Vertreter, ausgewählt aus Natriumalkoxid, Kaliumalkoxid, Magnesiumoxid, Calciumoxid, Natriumcarbonat, Kaliumcarbonat, Lithiumcarbonat, Cäsiumcarbonat, Calciumcarbonat, Natriumacetat, Kaliumacetat, Lithiumacetat, Natriumbenzoat, Kaliumbenzoat, Lithiumbenzoat, Triethylamin, Trimethylamin, Diisopropylethylamin, 1,8-Diazabicyclo[5.4.0]undec-7-en, 1,5-Diazabicyclo[4.3.0]non-5-en und Triethylendiamin und vorzugsweise ein Vertreter oder zwei oder mehr Vertreter, ausgewählt aus Natrium-tert-butoxid, Kaliumtert-butoxid, Natriumcarbonat, Kaliumcarbonat, Natriumacetat, Kaliumacetat, Triethylamin und Diisopropylethylamin, ist,
die Säure ein Vertreter oder zwei oder mehr Vertreter, ausgewählt aus Essigsäure, Benzoesäure, Salzsäure, Schwefelsäure, Phosphorsäure, Benzolsulfonsäure, p-Toluolsulfonsäure, Camphersulfonsäure, Bortrifluoridethylether, Scandiumtrifluormethansulfonat, Indiumtrifluormethansulfonat und Bismuttrifluormethansulfonat, und vorzugsweise ein Vertreter oder zwei oder mehr Vertreter, ausgewählt aus Essigsäure, p-Toluolsulfonsäure, Bortrifluorethylether und Bismuttrifluormethansulfonat, ist und
das Trockenmittel ein Vertreter oder zwei oder mehr Vertreter, ausgewählt aus Trockenmitteln, wie einem Molekularsieb, Magnesiumsulfat, Natriumsulfat und Calciumhydrid, und vorzugsweise ein Vertreter oder zwei oder mehr Vertreter, ausgewählt aus einem Molekularsieb und Magnesiumsulfat, ist.

4. Herstellungsverfahren gemäß Anspruch 1, worin das Molverhältnis des Hydrazins oder seines Derivats R₄NHNH₂ zu dem langkettigen Cyclohexenon-Alkohol-Rohprodukt III 0,8:1 bis 3:1 und vorzugsweise 0,9:1 bis 2:1 beträgt.

5. Herstellungsverfahren gemäß Anspruch 1, worin
die Kondensationsreaktion in einem Lösungsmittel durchgeführt wird,
das Lösungsmittel ein Vertreter oder zwei oder mehr Vertreter, ausgewählt aus Methanol, Ethanol, Isopropanol, n-Butanol, tert-Butanol, tert-Pentanol, Acetonitril, Tetrahydrofuran, Methyl-tert-butylether, Isopropyl Ether, Dioxan, Aceton, 2-Butanon, Ethylacetat, Isobutylacetat, Toluol, Xylol, Chlorbenzol, Benzol, N,N-Dimethylacetamid, N,N-Dimethylformamid, N,N-Diethylformamid, N-Methylpyrrolidon, Dichlormethan, 1,2-Dichlorethan, Chloroform, n-Hexane, n-Heptan, Cyclohexan und Wasser, und vorzugsweise ein Vertreter oder zwei oder mehr Vertreter, ausgewählt aus Methanol, Ethanol, Tetrahydrofuran, Acetonitril und n-Heptan, ist,
die Temperatur der Kondensationsreaktion 0 bis 149°C und vorzugsweise 20 bis 129°C beträgt und
Reaktionszeit der Kondensationsreaktion 0,5 bis 24 Stunden und vorzugsweise 1 bis 10 Stunden beträgt.

6. Herstellungsverfahren gemäß Anspruch 1, worin
die in Schritt (1b) genannte saure Substanz ein Vertreter oder zwei oder mehr Vertreter von einer organischen Säure, einer anorganischen Säure, einer Lewis-Säure, einem Säuresalz und einer anderen sauren Substanz ist,
die anorganische Säure Schwefelsäure, Salzsäure, Phosphorsäure, Polyphosphorsäure oder Phosphorwolframsäure ist,
die organische Säure Ameisensäure, Essigsäure, Propionsäure, Oxalsäure, Fumarsäure, Maleinsäure, Trifluoressigsäure, Methansulfonsäure, Benzolsulfonsäure, p-Toluolsulfonsäure, Camphersulfonsäure oder Trifluormethansulfonsäure ist,
die Lewis-Säure Bortrifluoridethylether, Aluminiumtrichlorid, Eisentrichlorid, Bismuttrifluormethansulfonat oder Scandiumtrifluormethansulfonat ist,
das Säuresalz ein Säuresalz, wie Natriumhydrogensulfat, Ammoniumhydrogensulfat, Magnesiumhydrogensulfat, Pyridinium-p-toluolsulfonat, Triethylaminhydrochlorid und Pyridinhydrochlorid ist,
die andere saure Substanz Silikagel oder saures Harz ist,
die saure Substanz vorzugsweise p-Toluolsulfonsäure, Salzsäure, Schwefelsäure, Natriumhydrogensulfat oder Magnesiumhydrogensulfat ist und
das Molverhältnis der hinzugefügten Menge der Verbindung II zu der hinzugefügten Menge der sauren Substanz 1:0,2 bis 1:10 und vorzugsweise 1:0,2 bis 1:2 beträgt.

7. Herstellungsverfahren gemäß Anspruch 1, worin
die Hydrolysereaktion in einem Lösungsmittel durchgeführt wird,
das Lösungsmittel ein Vertreter oder zwei oder mehr Vertreter, ausgewählt aus Benzol, Toluol, Chlorbenzol, Xylol, Acetonitril, 2-Butanon, Aceton, 1,2-Dimethyl-2-imidazolon, Dimethylsulfoxid, Dimethylsulfon, Sulfolan, Hexamethylphosphorsäuretriamid, N,N-Dimethylformamid, N,N-Dimethylacetamid, N,N-Diethylformamid, N-Methylpyrrolidon, Methanol, Ethanol, Isopropanol, n-Butanol, Ethylenglykol, Polyethylenglykol, Dioxan, Methyl-tert-butylether, Isopropylether, Tetrahydrofuran, n-Hexan, Cyclohexan, Dichlormethan, 1,2-Dichlorethan, Chloroform und Wasser ist und das Lösungsmittel vorzugsweise ein Vertreter oder zwei oder mehr Vertreter, ausgewählt aus Toluol, Acetonitril, Methanol, Ethanol, Wasser, Tetrahydrofuran, Methyl-tert-butylether und Dichlormethan, ist,
die Reaktionstemperatur der Hydrolysereaktion 20 bis 139°C beträgt und die Reaktionszeit der Hydrolysereaktion 0,5 bis 24 Stunden beträgt und
vorzugsweise die Reaktionstemperatur der Hydrolysereaktion 20 bis 100°C beträgt und die Reaktionszeit der Hydrolysereaktion 0,5 bis 10 Stunden beträgt.

8. Verfahren zur Herstellung eines durch Formel I dargestellten hochreinen langkettigen Cyclohexenon-Alkohols, erzielt durch das folgende Reaktionsschema: 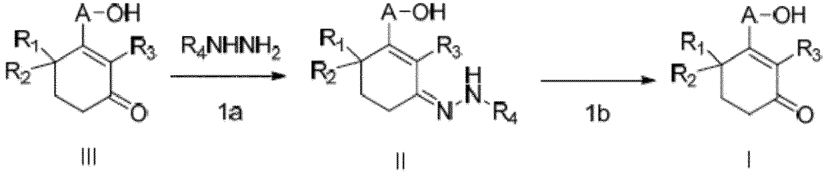 worin
A C₁₀₋₁₈-Alkylen darstellt,
R₁, R₂ und R₃ jeweils unabhängig H oder Methyl darstellen und
R₄ H, substituiertes oder unsubstituiertes C₁₋₇-Alkyl, substituiertes oder unsubstituiertes C₆₋₁₄-Aryl, 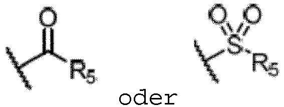 darstellt,
wobei "substituiert" substituiert mit einem Substituenten oder zwei oder mehr Substituenten, ausgewählt aus der Gruppe, bestehend aus Methyl, Nitro, Chlor und Brom, bedeutet, R₅ H, Methoxy, tert-Butoxy, Benzyloxy, Phenyl, 4-Methylphenyl oder Amino darstellt, und R₄ vorzugsweise 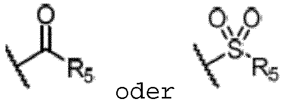 darstellt,
wobei das Verfahren die Schritte (1a)
Unterziehen eines langkettigen Cyclohexenon-Alkohol-Rohprodukts III und Hydrazin oder seinem Derivat R₄NHNH₂ einer Kondensationsreaktion, um eine Verbindung II zu erhalten, und
(1b) Hydrolysieren der Verbindung II in Gegenwart einer sauren Substanz, um die hochreine Verbindung I zu erhalten,
umfasst,
wobei das langkettige Cyclohexenon-Alkohol-Rohprodukt III durch das folgende Reaktionsschema erhalten wird: 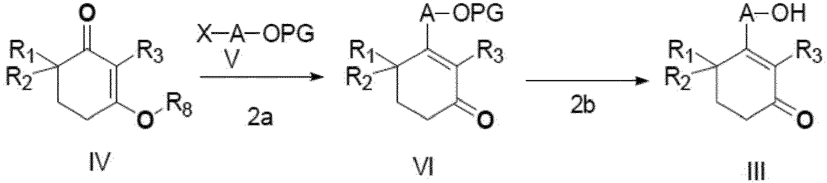
worin X Halogen darstellt, R₃ C₁₋₇-Alkyl, C₆₋₁₄-Aryl oder 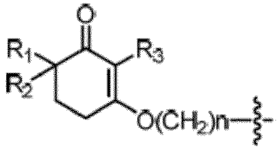 darstellt,
worin n 1 bis 12 darstellt,
PG 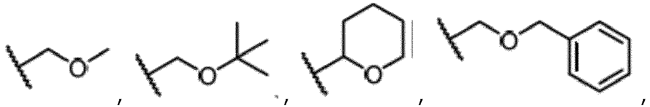 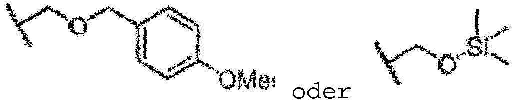 darstellt
und PG vorzugsweise 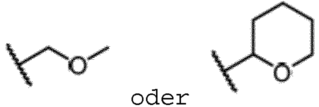 darstellt,
wobei das Verfahren die Schritte
(2a) Unterziehen einer Verbindung IV und einer Verbindung V einer metallvermittelten Barbier-Reaktion, um eine Verbindung VI zu erzeugen, und
(2b) Unterziehen der Verbindung VI einer Entschützungsreaktion in Gegenwart einer sauren Substanz zur Entfernung einer Schutzgruppe, wodurch das langkettige Cyclohexenon-Alkohol-Rohprodukt III erhalten wird,
umfasst.

9. Herstellungsverfahren gemäß Anspruch 8, worin in Schritt (2a)
das Metall Lithium, Natrium, Strontium, Magnesium oder Zink und vorzugsweise Lithium, Strontium oder Magnesium ist,
das Molverhältnis des Metalls zu der Verbindung IV 1:1 bis 12:1 und vorzugsweise 2:1 bis 10:1 beträgt und
das Molverhältnis der Verbindung V zu der Verbindung IV 0,6:1 bis 6:1 und vorzugsweise 0,8:1 bis 4:1 beträgt.

10. Herstellungsverfahren gemäß Anspruch 8, worin
die Barbier-Reaktion in Gegenwart oder Abwesenheit eines Katalysators durchgeführt wird,
der Katalysator ein Vertreter oder zwei oder mehr Vertreter, ausgewählt aus Tetramethylethylendiamin und Hexamethylphosphorsäuretriamid, ist,
das Molverhältnis des Katalysators zu der Verbindung IV 0,2:1 bis 2:1 und vorzugsweise 0,4:1 bis 1,2:1 beträgt,
die Barbier-Reaktion in einem geeigneten Lösungsmittel durchgeführt wird,
das Lösungsmittel ein Vertreter oder zwei oder mehr Vertreter, ausgewählt aus Benzol, Toluol, Chlorbenzol, Xylol, Tetrahydrofuran, Methyltetrahydrofuran, Dioxan, Methyl-tert-butylether, n-Hexan, n-Heptan, Cyclohexan, Acetonitril, Hexamethylphosphorsäuretriamid und Sulfolan, und vorzugsweise ein Vertreter oder zwei oder mehr Vertreter von Toluol, Xylol, Tetrahydrofuran, Methyltetrahydrofuran und n-Hexan ist,
die Temperatur der Barbier-Reaktion aus -20 bis 100°C und vorzugsweise -10 bis 50°C ausgewählt wird und
die Reaktionszeit der Barbier-Reaktion 1 bis 36 Stunden, vorzugsweise 2 bis 24 Stunden beträgt.

11. Herstellungsverfahren gemäß Anspruch 8, worin in Schritt (2b)
die saure Substanz ein Vertreter oder zwei oder mehr Vertreter von Methansulfonsäure, Benzolsulfonsäure, p-Toluolsulfonsäure, Camphersulfonsäure, Pyridinium-p-toluolsulfonat, Triethylaminhydrochlorid, Salzsäure, Schwefelsäure, Phosphorsäure, Natriumhydrogensulfat, Magnesiumhydrogensulfat, einem sauren Molekularsieb, saurem Harz, Essigsäure, Trifluoressigsäure, Trifluormethansulfonsäure, Eisentrichlorid, Bortrifluoridethylether und Acetylchlorid, und vorzugsweise ein Vertreter oder zwei oder mehr Vertreter von Benzolsulfonsäure, p-Toluolsulfonsäure, Camphersulfonsäure, Pyridinium p-toluolsulfonat, Salzsäure und Essigsäure ist,
das Molverhältnis der sauren Substanz zu der Verbindung VI 0,02:1 bis 1:1 und vorzugsweise 0,05:1 bis 0,2:1 beträgt,
die Entschützungsreaktion in einem geeigneten Lösungsmittel durchgeführt wird und das Lösungsmittel ein Vertreter oder zwei oder mehr Vertreter von Methanol, Ethanol, Isopropanol, n-Butanol, tert-Butanol, tert-Pentanol, Acetonitril, Tetrahydrofuran, Methyl-tert-butylether, Isopropylether, Dioxan, Aceton, 2-Butanon, Ethylacetat, Isobutylacetat, Toluol, Xylol, Chlorbenzol, Benzol, N,N-Dimethylacetamid, N,N-Dimethylformamid, N,N-Diethylformamid, N-Methylpyrrolidon, Dichlormethan, 1,2-Dichlorethan, Chloroform, n-Hexan, n-Heptan, Cyclohexan und Wasser und vorzugsweise ein Vertreter oder zwei oder mehr Vertreter von Methanol, Ethanol, Tetrahydrofuran, Acetonitril, n-Heptan und Wasser ist,
die Reaktionstemperatur der Entschützungsreaktion -20 bis 100°C und vorzugsweise 0 bis 50°C beträgt,
die Reaktionszeit der Entschützungsreaktion 0,1 bis 10 Stunden und vorzugsweise 0,5 bis 5 Stunden beträgt.

12. Herstellungsverfahren gemäß Anspruch 8, worin Schritt (2a) und Schritt (2b) getrennt schrittweise durchgeführt werden können oder durch ein Eintopfreaktionsverfahren durchgeführt werden können.

13. Verfahren zur Herstellung eines durch Formel I dargestellten hochreinen langkettigen Cyclohexenon-Alkohols, erzielt durch das folgende Reaktionsschema: 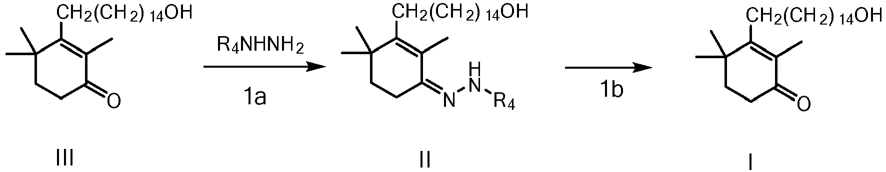 worin
A C₁₀₋₁₈-Alkylen darstellt,
R₁, R₂ und R₃ jeweils unabhängig H oder Methyl darstellen und
R₄ H, substituiertes oder unsubstituiertes C₁₋₇-Alkyl, substituiertes oder unsubstituiertes C₆₋₁₄-Aryl, 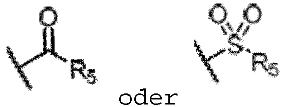 darstellt,
wobei "substituiert" substituiert mit einem Substituenten oder zwei oder mehr Substituenten, ausgewählt aus der Gruppe, bestehend aus Methyl, Nitro, Chlor und Brom, bedeutet, R₅ H, Methoxy, tert-Butoxy, Benzyloxy, Phenyl, 4-Methylphenyl oder Amino darstellt, und R₄ vorzugsweise 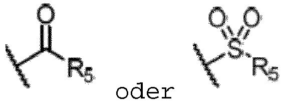 darstellt,
wobei das Verfahren die Schritte
(1a) Unterziehen eines langkettigen Cyclohexenon-Alkohol-Rohprodukts III und Hydrazin oder seinem Derivat R₄NHNH₂ einer Kondensationsreaktion, um eine Verbindung II zu erhalten, und
(1b) Hydrolysieren der Verbindung II in Gegenwart einer sauren Substanz, um die hochreine Verbindung I zu erhalten,
umfasst,
wobei das langkettige Cyclohexenon-Alkohol-Rohprodukt III durch das folgende Reaktionsschema erhalten wird: 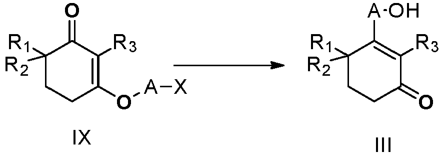
wobei eine Verbindung IX einer metallvermittelten intermolekularen Barbier-Reaktion unterzogen wird, um das langkettige Cyclohexenon-Alkohol-Rohprodukt II zu erhalten, R₁, R₂ und R₃ wie oben definiert sind, X Halogen darstellt und A C₁₀₋₁₈-Alkylen darstellt.

14. Herstellungsverfahren gemäß Anspruch 13, worin
das Metall Lithium, Natrium, Strontium, Magnesium oder Zink und vorzugsweise Lithium, Strontium oder Magnesium ist und
Molverhältnis des Metalls zu der Verbindung IX 1:1 bis 12:1 und vorzugsweise 2:1 bis 10:1 beträgt.

15. Herstellungsverfahren gemäß Anspruch 13, worin
die Barbier-Reaktion in Gegenwart oder Abwesenheit eines Katalysators durchgeführt wird,
der Katalysator ein Vertreter oder zwei oder mehr Vertreter, ausgewählt aus Tetramethylethylendiamin und Hexamethylphosphorsäuretriamid, ist,
das Molverhältnis des Katalysators zu der Verbindung IX 0,2 bis 2:1 und vorzugsweise 0,4 bis 1,2:1 beträgt,
die Barbier-Reaktion in einem geeigneten Lösungsmittel durchgeführt wird und das Lösungsmittel ein Vertreter oder zwei oder mehr Vertreter, ausgewählt aus Benzol, Toluol, Chlorbenzol, Xylol, Tetrahydrofuran, Methyltetrahydrofuran, Dioxan, Methyl-tert-butylether, n-Hexan, n-Heptan, Cyclohexan, Acetonitril, Hexamethylphosphorsäuretriamid und Sulfolan, und vorzugsweise ein Vertreter oder zwei oder mehr Vertreter von Toluol, Xylol, Tetrahydrofuran, Methyltetrahydrofuran und n-Hexan ist,
die Reaktionstemperatur der Barbier-Reaktion -20 bis 100°C und vorzugsweise -10 bis 50°C beträgt und
die Reaktionszeit der Barbier-Reaktion 1 bis 36 Stunden und vorzugsweise 2 bis 24 Stunden beträgt.

16. [3-(15-Hydroxypentadecyl)-2,4,4-trimethylcyclohex-2-en]-aminoformylhydrazon

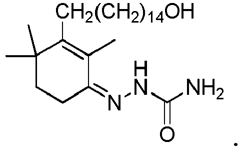

17. Verbindung, dargestellt durch Formel IX 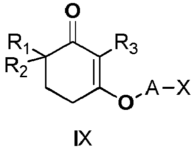 worin R₁, R₂ und R₃ jeweils unabhängig H oder Methyl darstellen, X Halogen darstellt und A C₁₀₋₁₈-Alkylen darstellt.

18. Verbindung gemäß Anspruch 17, die eine, ausgewählt aus 3-(15-Chlorpentadecyloxy-2,6,6-trimethylcyclohex-2-en-1-on) 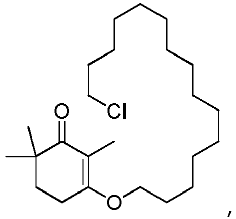 3-(15-Brompentadecyloxy)-2,6,6-trimethylcyclohex-2-en-1-on 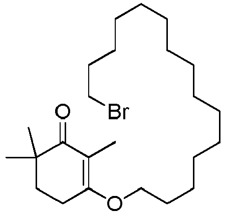 und
3 -(15-Iodpentadecyloxy)-2,6,6-trimethylcyclohex-2-en-1-on 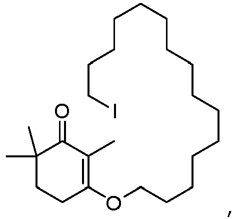 ist.

19. Verwendung der Verbindung gemäß Anspruch 17 oder 18 zur Herstellung einer durch Formel III dargestellten Verbindung, wie in Anspruch 1 definiert.

20. Verwendung der Verbindung gemäß Anspruch 16 zur Herstellung einer durch Formel I dargestellten Verbindung, wie in Anspruch 1 definiert.

21. 3-(15-Methoxymethylenoxy-pentadecyl)-2,4,4-trimethylcyclohex-2-en-1-on

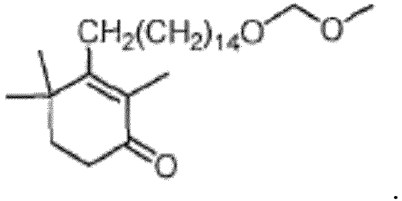

## Revendications

1. Procédé de production d'un alcool à longue chaîne de cyclohexénone de grande pureté représenté par la formule I, réalisé par le schéma réactionnel suivant : 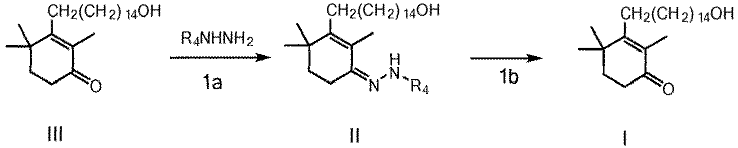 dans lequel
R₄ représente 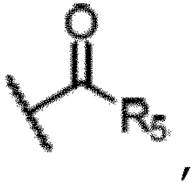
R₅ représente un amino,
le procédé comprenant les étapes consistant à
(la) soumettre un produit brut III d'alcool à longue chaîne de cyclohexénone et un dérivé d'hydrazine R₄NHNH₂ à une réaction de condensation pour obtenir un composé II, et
(1b) hydrolyser le composé II en présence d'une substance acide pour obtenir le composé I de grande pureté.

2. Procédé de production selon la revendication 1, dans lequel l'alcool à longue chaîne de cyclohexénone de grande pureté représenté par la formule I présente une pureté par HPLC supérieure à 95 % ; de préférence, l'alcool à longue chaîne de cyclohexénone de grande pureté représenté par la formule I présente une pureté par HPLC supérieure à 99 % ; et plus préférentiellement, l'alcool à longue chaîne de cyclohexénone de grande pureté représenté par la formule I présente une pureté par HPLC supérieure à 99,9 %.

3. Procédé de production selon la revendication 1, dans lequel
l'étape (1a) est effectuée en présence d'un acide, d'un alcalin, ou d'un déshydratant ;
l'alcalin est un élément ou deux éléments ou plus choisis parmi l'alcoxyde de sodium, l'alcoxyde de potassium, l'oxyde de magnésium, l'oxyde de calcium, le carbonate de sodium, le carbonate de potassium, le carbonate de lithium, le carbonate de césium, le carbonate de calcium, l'acétate de sodium, l'acétate de potassium, l'acétate de lithium, le benzoate de sodium, le benzoate de potassium, le benzoate de lithium, la triéthylamine, la triméthylamine, la diisopropyléthylamine, le 1,8-diazabicyclo[5,4,0]undec-7-ène, le 1,5-diazabicyclo[4,3,0]non-5-ène et la triéthylènediamine, et de préférence un élément ou deux éléments ou plus choisis parmi le tert-butoxyde de sodium, le tert-butoxyde de potassium, le carbonate de sodium, le carbonate de potassium, l'acétate de sodium, l'acétate de potassium, la triéthylamine et la diisopropyléthylamine ;
l'acide est un élément ou deux éléments ou plus choisis parmi l'acide acétique, l'acide benzoïque, l'acide chlorhydrique, l'acide sulfurique, l'acide phosphorique, l'acide benzènesulfonique, l'acide p-toluènesulfonique, l'acide camphorsulfonique, l'éther éthylique de trifluorure de bore, le trifluorométhanesulfonate de scandium, le trifluorométhanesulfonate d'indium et le trifluorométhanesulfonate de bismuth, et de préférence un élément ou deux éléments ou plus choisis parmi l'acide acétique, l'acide p-toluènesulfonique, l'éther éthylique de trifluorure de bore et le trifluorométhanesulfonate de bismuth ; et
le déshydratant est un élément ou deux éléments ou plus choisis parmi des déshydratants, tels qu'un tamis moléculaire, le sulfate de magnésium, le sulfate de sodium et l'hydrure de calcium, et de préférence un élément ou deux éléments ou plus choisis parmi un tamis moléculaire et le sulfate de magnésium.

4. Procédé de production selon la revendication 1, dans lequel le rapport molaire de l'hydrazine ou de son dérivé R₄NHNH₂ sur le produit brut III d'alcool à longue chaîne de cyclohexénone va de 0,8/1 à 3/1, et de préférence de 0,9/1 à 2/1.

5. Procédé de production selon la revendication 1, dans lequel
la réaction de condensation est effectuée dans un solvant ;
le solvant est un élément ou deux éléments ou plus choisis parmi le méthanol, l'éthanol, l'isopropanol, le n-butanol, le tert-butanol, le tert-pentanol, l'acétonitrile, le tétrahydrofurane, le méthyl tert-butyl éther, l'éther isopropylique, le dioxane, l'acétone, le 2-butanone, l'acétate d'éthyle, l'acétate d'isobutyle, le toluène, le xylène, le chlorobenzène, le benzène, le N,N-diméthylacétamide, le N,N-diméthylformamide, le N,N-diéthylformamide, le N-méthylpyrrolidone, le dichlorométhane, le 1,2-dichloroéthane, le chloroforme, le n-hexane, le n-heptane, le cyclohexane et l'eau, et de préférence un élément ou deux éléments ou plus choisis parmi le méthanol, l'éthanol, le tétrahydrofurane, l'acétonitrile et le n-heptane ;
la température de la réaction de condensation va de 0 à 149 °C, et de préférence de 20 à 129 °C ; et
le temps de réaction de la réaction de condensation va de 0,5 à 24 heures, et de préférence de 1 à 10 heures.

6. Procédé de production selon la revendication 1, dans lequel
la substance acide énumérée à l'étape (1b) est un élément ou deux éléments ou plus parmi un acide organique, un acide inorganique, un acide de Lewis, un sel d'acide et une autre substance acide ;
l'acide inorganique est l'acide sulfurique, l'acide chlorhydrique, l'acide phosphorique, l'acide polyphosphorique ou l'acide phosphotungstique ;
l'acide organique est l'acide formique, l'acide acétique, l'acide propionique, l'acide oxalique, l'acide fumarique, l'acide maléique, l'acide trifluoroacétique, l'acide méthanesulfonique, l'acide benzènesulfonique, l'acide p-toluènesulfonique, l'acide camphorsulfonique ou l'acide trifluorométhanesulfonique ;
l'acide de Lewis est l'éther éthylique de trifluorure de bore, le trichlorure d'aluminium, le trichlorure de fer, le trifluorométhanesulfonate de bismuth ou le trifluorométhanesulfonate de scandium ;
le sel d'acide est un sel d'acide, tel que l'hydrogénosulfate de sodium, l'hydrogénosulfate d'ammonium, l'hydrogénosulfate de magnésium, le p-toluènesulfonate de pyridinium, le chlorhydrate de triéthylamine et le chlorhydrate de pyridine ;
l'autre substance acide est le gel de silice ou la résine acide ;
de préférence la substance acide est l'acide p-toluènesulfonique, l'acide chlorhydrique, l'acide sulfurique, l'hydrogénosulfate de sodium ou l'hydrogénosulfate de magnésium ; et
le rapport molaire de la quantité ajoutée du composé II sur la quantité ajoutée de la substance acide va de 1/0,2 à 1/10, et de préférence de 1/0,2 à 1/2.

7. Procédé de production selon la revendication 1, dans lequel
la réaction d'hydrolyse est effectuée dans un solvant ;
le solvant est un élément ou deux éléments ou plus choisis parmi le benzène, le toluène, le chlorobenzène, le xylène, l'acétonitrile, le 2-butanone, l'acétone, le 1,2-diméthyl-2-imidazolone, le diméthylsulfoxyde, la diméthylsulfone, le sulfolane, le triamide hexaméthylphosphorique, le N,N-diméthylformamide, le N,N-diméthylacétamide, le N,N-diéthylformamide, le N-méthylpyrrolidone, le méthanol, l'éthanol, l'isopropanol, le n-butanol, l'éthylène glycol, le polyéthylène glycol, le dioxane, le méthyl tert-butyl éther, l'éther isopropylique, le tétrahydrofurane, le n-hexane, le cyclohexane, le dichlorométhane, le 1,2-dichloroéthane, le chloroforme et l'eau, et de préférence le solvant est un élément ou deux éléments ou plus choisis parmi le toluène, l'acétonitrile, le méthanol, l'éthanol, l'eau, le tétrahydrofurane, le méthyl tert-butyl éther et le dichlorométhane ;
la température de réaction de la réaction d'hydrolyse va de 20 à 139 °C, et le temps de réaction de la réaction d'hydrolyse va de 0,5 à 24 heures, et
de préférence la température de réaction de la réaction d'hydrolyse va de 20 à 100 °C, et le temps de réaction de la réaction d'hydrolyse va de 0,5 à 10 heures.

8. Procédé de production d'un alcool à longue chaîne de cyclohexénone de grande pureté représenté par la formule I, réalisé par le schéma réactionnel suivant : 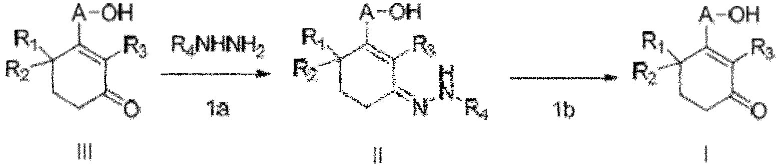 dans lequel
A représente un alkylène en C_{10 à 18} ;
R₁, R₂ et R₃ représentent chacun indépendamment H ou un méthyle ; et
R₄ représente H, un alkyle en C_{1 à 7} substitué ou non substitué, un aryle en C_{6 à 14} substitué ou non substitué, 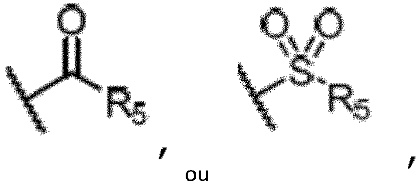
dans lequel « substitué » signifie substitué par un substituant ou deux substituants ou plus choisis parmi un méthyle, un nitro, un chlore et un brome ; R₅ représente H, un méthoxy, un tert-butoxy, un benzyloxy, un phényle, un 4-méthylphényle ou un amino ; et R₄ représente de préférence 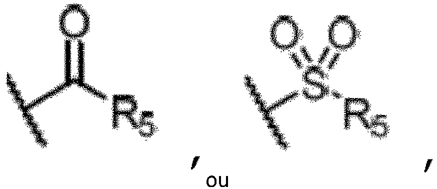
le procédé comprenant les étapes consistant à
(la) soumettre un produit brut III d'alcool à longue chaîne de cyclohexénone et l'hydrazine ou son dérivé R₄NHNH₂ à une réaction de condensation pour obtenir un composé II, et
(1b) hydrolyser le composé II en présence d'une substance acide pour obtenir le composé I de grande pureté,
dans lequel le produit brut III d'alcool à longue chaîne de cyclohexénone est obtenu par le schéma réactionnel suivant : 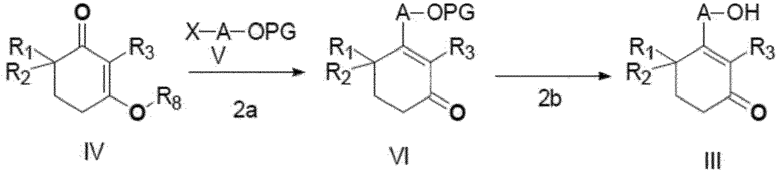
dans lequel X représente un halogène, R₈ représente un alkyle en C_{1 à 7}, un aryle en C₆ à ₁₄, ou 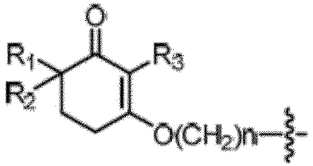
dans lequel n vaut de 1 à 12,
PG représente 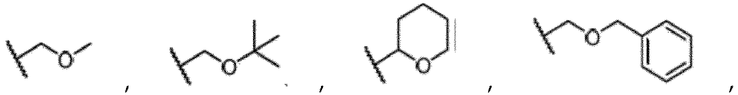 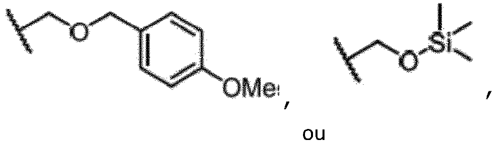
et PG représente de préférence 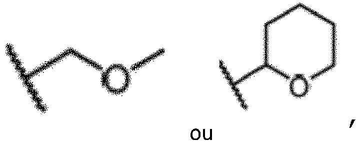
le procédé comprenant les étapes consistant à
(2a) soumettre un composé IV et un composé V à une réaction de Barbier induite par un métal pour produire un composé VI, et
(2b) soumettre le composé VI à une réaction de déprotection en présence d'une substance acide pour éliminer un groupe protecteur, permettant ainsi d'obtenir le produit brut III d'alcool à longue chaîne de cyclohexénone.

9. Procédé de production selon la revendication 8, dans lequel à l'étape (2a),
le métal est le lithium, le sodium, le strontium, le magnésium ou le zinc, et de préférence le lithium, le strontium ou le magnésium,
le rapport molaire du métal sur le composé IV va de 1/1 à 12/1, et de préférence de 2/1 à 10/1, et
le rapport molaire du composé V sur le composé IV va de 0,6/1 à 6/1, et de préférence de 0,8/1 à 4/1.

10. Procédé de production selon la revendication 8, dans lequel
la réaction de Barbier est effectuée en présence ou en l'absence d'un catalyseur,
le catalyseur est un élément ou deux éléments ou plus choisis parmi le tétraméthyléthylènediamine et le triamide hexaméthylphosphorique,
le rapport molaire du catalyseur sur le composé IV va de 0,2/1 à 2/1, et de préférence de 0,4/1 à 1,2/1,
la réaction de Barbier est effectuée dans un solvant approprié,
le solvant est un élément ou deux éléments ou plus choisis parmi le benzène, le toluène, le chlorobenzène, le xylène, le tétrahydrofurane, le méthyltétrahydrofurane, le dioxane, le méthyl tert-butyl éther, le n-hexane, le n-heptane, le cyclohexane, l'acétonitrile, le triamide hexaméthylphosphorique et le sulfolane, et de préférence un élément ou deux éléments ou plus choisis parmi le toluène, le xylène, le tétrahydrofurane, le méthyltétrahydrofurane et le n-hexane,
la température de la réaction de Barbier est choisie de -20 à 100 °C, et de préférence de -10 à 50 °C, et
le temps de réaction de la réaction de Barbier va de 1 à 36 heures, et de préférence de 2 à 24 heures.

11. Procédé de production selon la revendication 8, dans lequel à l'étape (2b),
la substance acide est un élément ou deux éléments ou plus parmi l'acide méthanesulfonique, l'acide benzènesulfonique, l'acide p-toluènesulfonique, l'acide camphorsulfonique, le p-toluènesulfonate de pyridinium, le chlorhydrate de triéthylamine, l'acide chlorhydrique, l'acide sulfurique, l'acide phosphorique, l'hydrogénosulfate de sodium, l'hydrogénosulfate de magnésium, un tamis moléculaire acide, une résine acide, l'acide acétique, l'acide trifluoroacétique, l'acide trifluorométhanesulfonique, le trichlorure de fer, l'éther éthylique de trifluorure de bore et le chlorure d'acétyle, et de préférence un élément ou deux éléments ou plus parmi l'acide benzènesulfonique, l'acide p-toluènesulfonique, l'acide camphorsulfonique, le p-toluènesulfonate de pyridinium, l'acide chlorhydrique et l'acide acétique,
le rapport molaire de la substance acide sur le composé VI va de 0,02/1 à 1/1, et de préférence de 0,05/1 à 0,2/1,
la réaction de déprotection est effectuée dans un solvant approprié, et le solvant est un élément ou deux éléments ou plus parmi le méthanol, l'éthanol, l'isopropanol, le n-butanol, le tert-butanol, le tert-pentanol, l'acétonitrile, le tétrahydrofurane, le méthyl tert-butyl éther, l'éther isopropylique, le dioxane, l'acétone, le 2-butanone, l'acétate d'éthyle, l'acétate d'isobutyle, le toluène, le xylène, le chlorobenzène, le benzène, le N,N-diméthylacétamide, le N,N-diméthylformamide, le N,N-diéthylformamide, le N-méthylpyrrolidone, le dichlorométhane, le 1,2-dichloroéthane, le chloroforme, le n-hexane, le n-heptane, le cyclohexane et l'eau, et de préférence un élément ou deux éléments ou plus parmi le méthanol, l'éthanol, le tétrahydrofurane, l'acétonitrile, le n-heptane et l'eau,
la température de réaction de la réaction de déprotection va de -20 à 100 °C, et de préférence de 0 à 50 °C,
le temps de réaction de la réaction de déprotection va 0,1 à 10 heures, et de préférence de 0,5 à 5 heures.

12. Procédé de production selon la revendication 8, dans lequel l'étape (2a) et l'étape (2b) peuvent être effectuées séparément par étape, ou peuvent être effectuées par un procédé de réaction monotope.

13. Procédé de production d'un alcool à longue chaîne de cyclohexénone de grande pureté représenté par la formule I, réalisé par le schéma réactionnel suivant : 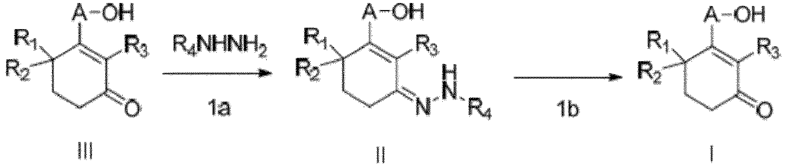 dans lequel
A représente un alkylène en C_{10 à 18;}
R₁, R₂ et R₃ représentent chacun indépendamment H ou un méthyle ; et
R₄ représente H, un alkyle en C_{1 à 7} substitué ou non substitué, un aryle en C_{6 à 14} substitué ou non substitué, 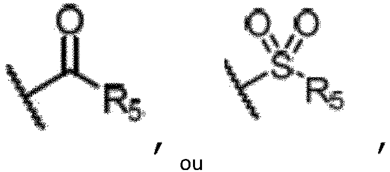
dans lequel « substitué » signifie substitué par un substituant ou deux substituants ou plus choisis parmi un méthyle, un nitro, un chlore et un brome ; R₅ représente H, un méthoxy, un tert-butoxy, un benzyloxy, un phényle, un 4-méthylphényle ou un amino ; et R₄ représente de préférence 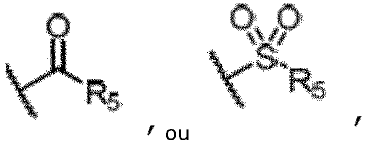
le procédé comprenant les étapes consistant à
(1a) soumettre un produit brut III d'alcool à longue chaîne de cyclohexénone et l'hydrazine ou son dérivé R₄NHNH₂ à une réaction de condensation pour obtenir un composé II, et
(1b) hydrolyser le composé II en présence d'une substance acide pour obtenir le composé I de grande pureté,
dans lequel le produit brut III d'alcool à longue chaîne de cyclohexénone est obtenu par le schéma réactionnel suivant : 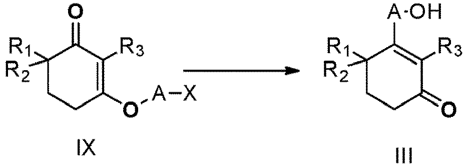
dans lequel un composé IX est soumis à une réaction de Barbier intermoléculaire induite par un métal pour obtenir le produit brut III d'alcool à longue chaîne de cyclohexénone, R₁, R₂ et R₃ sont tels que définis ci-dessus, X représente un halogène, et A représente un alkylène en C_{10 à 18}.

14. Procédé de production selon la revendication 13, dans lequel
le métal est le lithium, le sodium, le strontium, le magnésium ou le zinc, et de préférence le lithium, le strontium ou le magnésium, et
le rapport molaire du métal sur le composé IX va de 1/1 à 12/1, et de préférence de 2/1 à 10/1.

15. Procédé de production selon la revendication 13, dans lequel
la réaction de Barbier est effectuée en présence ou en l'absence d'un catalyseur,
le catalyseur est un élément ou deux éléments ou plus choisis parmi le tétraméthyléthylènediamine et le triamide hexaméthylphosphorique,
le rapport molaire du catalyseur sur le composé IX va de 0,2/1 à 2/1, et de préférence de 0,4/1 à 1,2/1,
la réaction de Barbier est effectuée dans un solvant approprié, et le solvant est un élément ou deux éléments ou plus choisis parmi le benzène, le toluène, le chlorobenzène, le xylène, le tétrahydrofurane, le méthyltétrahydrofurane, le dioxane, le méthyl tert-butyl éther, le n-hexane, le n-heptane, le cyclohexane, l'acétonitrile, le triamide hexaméthylphosphorique et le sulfolane, et de préférence un élément ou deux éléments ou plus choisis parmi le toluène, le xylène, le tétrahydrofurane, le méthyltétrahydrofurane et le n-hexane,
la température de la réaction de Barbier va de -20 à 100 °C, et de préférence de - 10 à 50 °C, et
le temps de réaction de la réaction de Barbier va de 1 à 36 heures, et de préférence de 2 à 24 heures.

16. [3-(15-hydroxypentadécyl)-2,4,4-triméthylcyclohex-2-ène]-aminoformylhydrazone

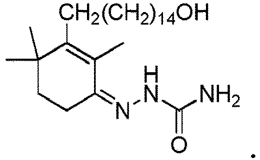

17. Composé représenté par la formule IX 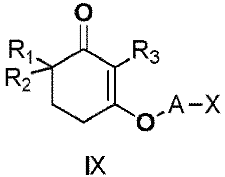 dans lequel R₁, R₂ et R₃ représentent chacun indépendamment H ou un méthyle, X représente un halogène, et A représente un alkylène en C₁₀ à ₁₈.

18. Composé selon la revendication 17, qui est un élément choisi parmi le 3-(15-chloropentadécyloxy-2,6,6-triméthylcyclohex-2-en-1-one) 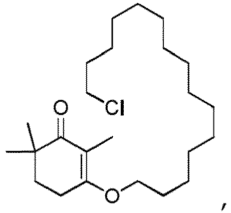 le 3-(15-bromopentadécyloxy-2,6,6-triméthylcyclohex-2-en-1-one) 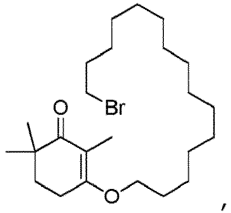 et
le 3-(15-iodopentadécyloxy)-2,6,6-triméthylcyclohex-2-en-1-one

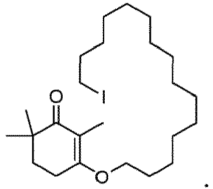

19. Utilisation du composé selon la revendication 17 ou 18 pour produire un composé représenté par la formule III tel que défini selon la revendication 1.

20. Utilisation du composé selon la revendication 16 pour produire un composé représenté par la formule I tel que défini selon la revendication 1.

21. 3-(15-méthoxyméthylèneoxy-pentadécyl)-2,4,4-triméthylcyclohex-2-en-1-one
